(19) Europäisches Patentamt
European Patent Office
Office européen des brevets

(11) EP 0 745 573 A1

(12) EUROPEAN PATENT APPLICATION
published in accordance with Art. 158(3) EPC

(43) Date of publication:
04.12.1996 Bulletin 1996/49

(51) Int. Cl.$^6$: C05F 11/08, C02F 11/02,
B09B 3/00, C12P 1/00

(21) Application number: 95903004.0

(22) Date of filing: 20.12.1994

(86) International application number:
PCT/JP94/02155

(87) International publication number:
WO 96/19420 (27.06.1996 Gazette 1996/29)

(84) Designated Contracting States:
AT CH DE ES FR GB IT LI NL

(71) Applicant: Kimura, Mitsuyo
Shinzyuku-ku, Toyo 160 (JP)

(72) Inventors:
• KIMURA, Mitsuyo
Tokyo 160 (JP)
• HIURA, Kouichiro
Shizuoka-ken 425 (JP)

• MAEDA, Tokihisa
Shizuoka-ken 425 (JP)

(74) Representative: Jones, Helen Marjorie Meredith
et al
Gill Jennings & Every,
Broadgate House,
7 Eldon Street
London EC2M 7LH (GB)

(54) FERMENTATION PRODUCT AND PROCESS FOR PRODUCING THE SAME

(57) A fermentation product having excellent water retentiveness and permeability and containing a slow-release fertilizer component is obtained by growing microorganisms which are viable in an alkaline environment sequentially and preferentially in accordance with the environment which varies with the lapse of time to thereby effect alkaline fermentation of the reaction product. The fermentation product is a product of reaction btween a putrefacient waste comprising a solidliquid mixture and additives mainly comprising highly active calcium oxide and contains microorganisms viable in a highly alkaline environment with a pH value of 12 or above. The microorganisms are composed of a bacterial group comprising first group bacteria which begins growing in a pH range from 11 to less than 12, second group bacteria which grows well in a pH range from about 11 to 9 and third group bacteria which grows well below a pH of about 9, fungi and bacterium 90A1. The pH of the fermentation product is maintained near 8 as a result of the bacteria growth.

FIG. 7

EP 0 745 573 A1

Printed by Rank Xerox (UK) Business Services
2.13.8/3.4

**Description**

FIELD OF THE INVENTION

This invention relates to a fermentative product treated from putrefactive wastes and method for the production, more particularly, to the fermentative product obtained through the fermentation in alkaline pH at a desired period with aids of specific microorganisms which have lived in status of durability after natural putrefaction of the waste had once been suspended and the method for the production.

BACKGROUND OF THE INVENTION

Cattle excreta, excess sludge from sewers and waterworks or any other putrefactive wastes (including residues from distilled spirits or animal bloods etc.,) have generally been converted to substances as composts or soil improving agents through decompositions of organic matters with aids of the microorganisms in the genus Enterobacteriaceae, e.g., E.coli or Enterobacteria surviving in the putrefactive waste or specific microorganisms e.g., photosynthesis bacteria or kouji-fungi which were added in the putrefactive waste.

However, techniques in the past for converting the putrefactive waste to the compost or the like through the fermentation have generally been regarded as ones relying on a method presented as a word "fermentation" but in fact have relied on the mere decomposition of the organic matters with aids of the microorganisms as mentioned above, i.e., natural putrefaction. The techniques, therefore, have problems in that stink is given off over a long period, soluble components dissolved in the putrefactive waste are flown away and the conversion of the wastes to the composts costs a long period of time and is difficult for human to control. Another problem is a second putrefaction which deteriorates the substance.

Of the substances thus obtained through the fermentation, the composts, when mixed into soil, sometimes cause phenomena such as starvation of nitrogen resulting from absorption of nitrogen by the microorganisms or over-richness of soil, to the contrary. Also, those named as the soil improving agents, most of which lack sufficient abilities of water retention or water permeation into soil or fail to obtain components effective as fertilizers. Therefore, they hardly give substantially preventive effect on disease.

It is an object of the present invention to solve the various problems in the fermentative product of putrefaction. In other words, the object of the present invention is to provide alkaline fermentative product obtained by sterilizing the putrefactive waste by the co-actions of high heat and a highly alkaline environment for a temporal suspension of progress in the putrefaction and causing sequential proliferations of the microorganisms which have been in status of durability under the sterile environment and can grow in highly alkaline condition and the method for the production.

It is another object of the present invention to provide the fermentative product which is fermented for short time, is easy for human to control and is freed from the potential second fermentation and the method for the production.

It is further object of the present invention to provide the fermentative product being excellent in the water permeability into the soil and the ability of the water retention, having the components as the fertilizers which are soluble into the soil by degrees and directly solely utilizable as a culture soil and the method for the production.

Furthermore, it is also an object of the present invention to provide the fermentative product effective for preventing disease and the method for the production.

DISCLOSURE OF THE INVENTION

The fermentative product according to the present invention is characterized by;

consisting of a reaction product obtained through a reaction between the putrefactive waste mixed with solid liquid and an additive whose main component is highly activated calcium oxide,

containing the microorganisms surviving under the highly alkaline environment with pH of 12 or more, the microorganisms consisting of bacteria in 3 groups, filamentous fungi and 90A1 bacterium, the first group of the bacteria starting its proliferation at pH of 11 to less than 12, the second group of the bacteria preferably growing at pH of around 11 to 9 and the third group of the bacteria preferably growing at ph of less than around 9,

and its pH being maintained around 8 through the proliferation of the microorganisms.

Also, the method for producing the fermentative product according to the present invention is characterized by;

causing a vigorous hydration reaction by adding the additive whose main component is highly activated quick lime to the putrefactive waste to suspend the putrefaction of the reaction product by the co-actions of the reaction heat generated from this hydration reaction and the alkaline sterilization under the highly alkaline environment, while allowing existence of the microorganisms in the reaction product which are detectable in status of durability and can grow in highly alkaline status, the microorganisms consisting of the bacteria in 3 groups, filamentous fungi and 90A1 bacterium, the first group of the bacteria starting its proliferation at pH of 11 to less than 12, the second group of the bacteria preferably growing at pH of around 11 to 9 and the third group of the bacteria preferably growing at pH of less than around 9,

and supplying water and air at a suitable period for the reaction product thereby utilizing the bacteria to bring about sequential decline in pH value to cause the proliferation of filamentous fungi and 90A1 bacterium which have survived in status of durability in the reaction product with the pH declination for the alkaline fermentation of the reaction product.

The method of the present invention is to ferment the putrefactive waste by utilizing the microorganisms which have been in status of durability under the alkaline sterile environments. One of the advantage of the present invention is, therefore, to allow the conversion of the putrefactive waste to the substance equivalent to matured composts at ease and low cost for short period of time.

Another advantage of the present invention is to allow start of the proliferation of the microorganisms under control by human. The fermentative product, therefore, is obtainable which can be under the control by human relative to the fermentation and is freed from the potential second fermentation.

Further, the fermentative product of the present invention is excellent in the water permeability into the soil or the ability of the water retention and have the components as the fertilizers which are gradually soluble into the soil and provided as the substance which is directly solely utilizable as the culture soil, due to the specific physical structure of the reaction product.

Furthermore, the present invention advantageously can provide the substance provided with functions of preventing various diseases by the microorganisms and the environment in which the microorganisms having specific physical structure are surviving.

BRIEF DESCRIPTION OF THE DRAWINGS

The present invention will be described in conjunction with accompanying drawings in which--

Fig. 1 is a graph showing the compared velocity of the hydration reaction of calcium oxide used for the present invention with that of quick lime on the market.

Fig. 2 is a graph showing the difference in the hydration reaction between calcium oxide used for the present invention and quick limes on the market, the difference being indicated at rates of experimental reaction temperatures to theoretical values.

Fig. 3 are graphs showing the compared rates of the dissolved organic matters, one showing the dissolution from the reaction product and the other from the control at times of water washing just after the hydration reaction of the present invention.

Fig. 4 is a graph showing a curve of adjusting ability of the variation of soil pH when the reaction product is used in the soil.

Fig. 5 is a graph showing a curve of pF-water in the reaction product.

Fig. 6 is a graph embodying the water movement when the reaction product was used in the soil.

Fig. 7 is a graph showing the variation of the numbered microorganisms in the fermentation.

Fig. 8 is a graph showing changes in physical properties of the reaction product along with the fermentation.

Fig. 9 is a drawing of arranged compartments for testing groups in the embodiment wherein the present fermentative product was supplied for rice fields.

Fig. 10 is a drawing of the arranged compartments for the testing groups in the embodiment showing the investigation into the amount of barley.

PREFERRED EMBODIMENTS

As raw materials of the present invention, employed putrefactive waste are the swine excreta (including the excrements), the chicken dung or any other livestock excreta. Besides, the animal blood, the excess sludge from the sewers and waterworks or debris of putrefaction such as the residue from the distilled spirits wasted from food manufacturing plants are employed. For example, normally contained water is in the range of 86.5% to 94.5% in the swine excreta, 15% to 30% in the dried chicken dung, 75% to 97% in the excess sludge from the sewers and waterworks and 75% to 95% in the residue of putrefaction from the foods manufacturing plants. Advantageously, the contained water is controlled to 75 to 97 % for used as the raw material of the present invention.

To the raw materials, added is the additive whose main component is the highly activated calcium oxide. Both are mixed and stirred up. More specifically, relative to 100 parts by weight of the industrial waste of putrefaction, 5 to 25 parts by weight of the pre-determined additive is added for causing a reaction therebetween.

Advantageously, the highly activated additive has conditions as follows;

① having high rate of containing calcium oxide (advantageously more than 95%) but being low in the rate of containing calcium hydroxide, calcium carbonate and any others. Magnesium oxide may be contained in little (e.g., below 5%) as one of the composite components.

② having porosity, wide surface area and specific surface area and highly developed pore organization.

③ having a property excellent in dispersion, e.g., widely quickly dispersing in every direction, at a time of contact in little with the water.

④ vigorously and quickly reacting, when added in medium amount to water, to produce steam.

⑤ fully reacting, when added in a certain amount to the water, to have the temperature raised which is recognized to be analogous to the theoretical value.

Further, if needed,

⑥ having such small settling velocity that the settling phenomena can not be recognized, when being in slurry whose main component is slaked lime after contact with the water.

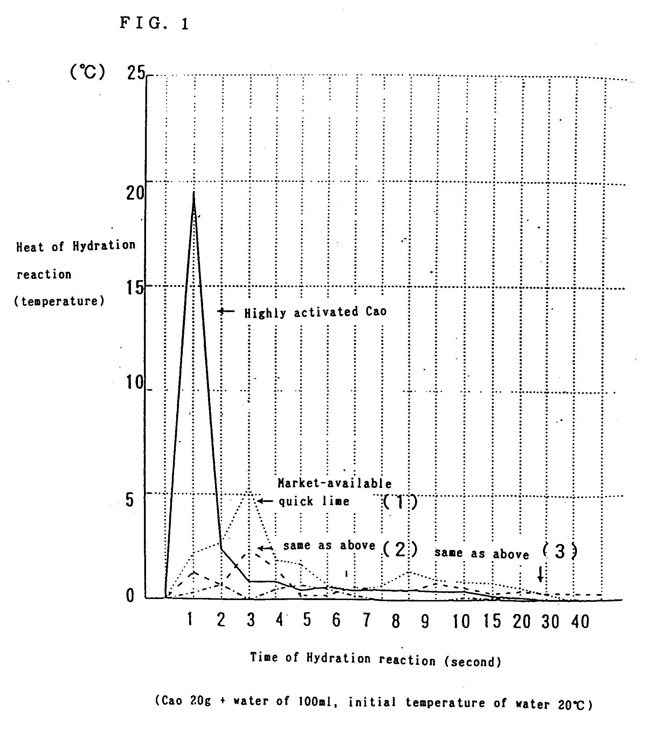

Fig. 1 shows the result of the compared velocity of the temperature-rising between the additive used in the present invention when added to the water and that of quick limes on the market.

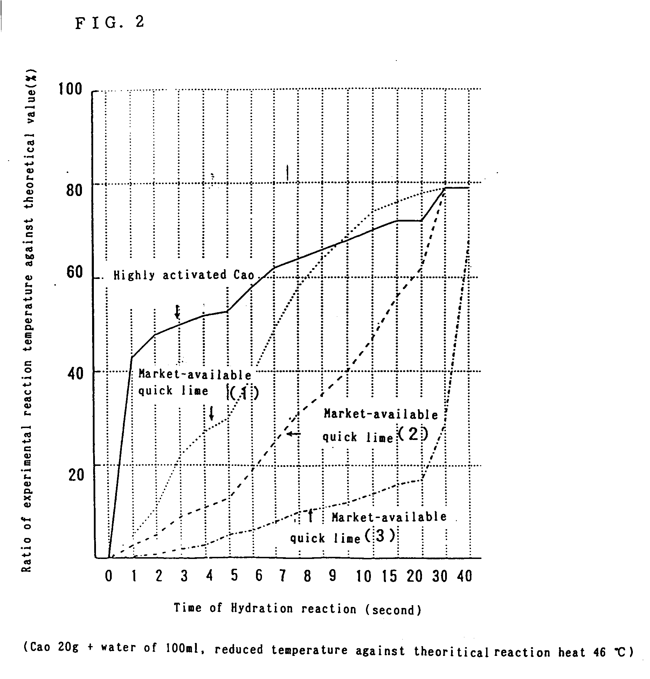

Fig. 2 shows the rates of the respective experimental reaction temperatures relative to four matters to the theoretical value at the time of the reaction. The matters include three kinds of quick limes on the market, each of which is; (1) one never in contact with air, just after seal-opened, (2) one left for an hour under circumstance of 90 % humidity, after seal-opened and (3) one left for 4 hours under the same circumstance, after seal-opened. Fig. 1 and 2 show the differences in the velocity of the temperature-rising (°C/second) and the rates of the experimental temperatures to the theoretical value, when the present additive and the three kinds of the limes on the market (1) to (3), each 20 g, are added to 100 ml water having initial temperature of 20°C.

As is evident from Fig. 1 and 2, it is found that the velocity of the temperature-rising of the present additive was significantly more than that of those on the market (1) to (3), more specifically, the temperature instantaneously has risen to a high point for very short period of time (around 1 second). This means that most of calcium are instantly ionized by the hydration reaction to be dissociated from calcium oxide and a local high-heat status is created which is necessary for thermally decomposing the organic matters contained in the putrefactive waste.

By addition of the present additive to the putrefactive waste, cellulose, lignin, high molecular weight protein and phospholipid are stimulated under the alkaline condition to be decomposed by the local high heat resulting from the reaction between calcium oxide and water into low molecular compounds. Then, calcium ion which was rapidly dissociated is connected to not only terminal radical of the decomposed low molecular compound, phospholipid as referred above and higher fatty acids but also lower fatty acids e.g., formic acid or acetic acid to produce hardly dissolved calcium salt in stabilized status in the reaction product. Calcium compound thus produced through the chemical reaction, especially through a complex salt reaction with lower fatty acids, is provided as one perfectly different from matters stabilized only in physical properties, the matters being produced through an aggregative action caused where, e.g., slaked lime or said quick limes on the market is added. In these cases, calcium ion is less dissociated or at slow speed with the organic matters in the putrefactive waste decomposed insufficiently. As a result, calcium salt can not be produced.

Tables 1 to 4 show respective changes in the amount of carbon, nitrogen, lipid, main organic acids and inorganic component contained in the livestock excreta, when added with parts of the present additive, each 5% (F5), 10% (F10) and 20% (F20). In these tables, the control is the raw swine excreta before the additive is added. Respective reduced values were obtained by multiplying the experimental value of each component in the treated matter added with the additive by (division of the amount of the organic matters in the control by that in the treated added with the additive). The decreased rate was obtained by a calculation based on the formula :1-(division of the reduced value of each component in the treated added with the additive by the experimental value of each component in the control) × 100 %.

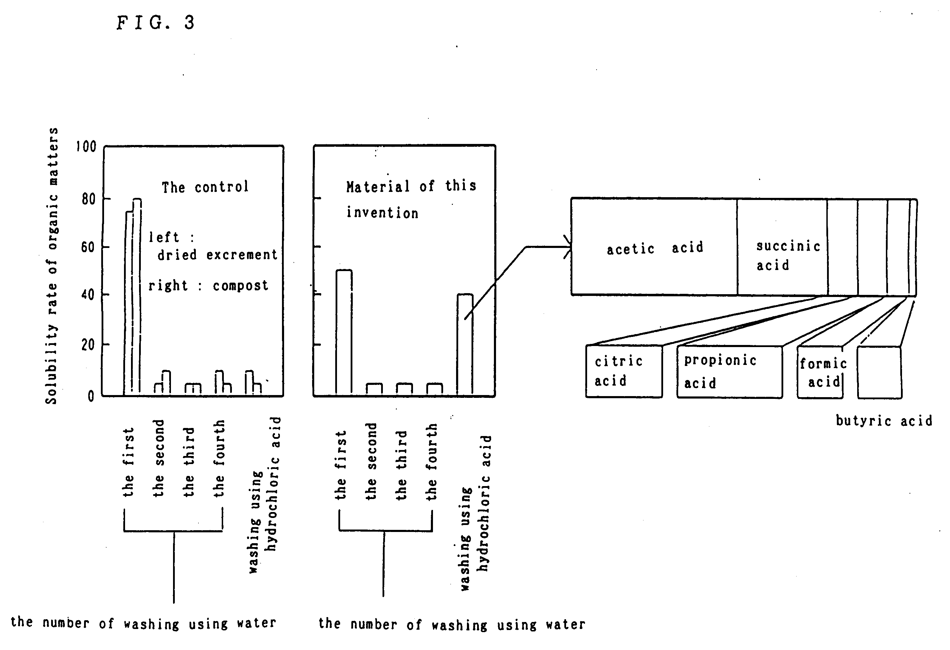

Fig. 3 are the graphs showing the result of the tests for suggesting that soluble organic acids contained in the putrefactive waste are fixed as hardly soluble calcium salt in the reaction product of the present invention.

Less than 10 % is indicated as a rate of the organic acids dissolved from the dried excreta and the composts at a hydrochloric acid cleaning after the water washing. In contrast, from the reaction product, the organic acids of as much as 40 % are dissolved even at the hydrochloric acid cleaning after the water washing. This suggests that the reaction product according to the present invention contains the organic acids which are fixed as calcium compound hardly dissolved in the water but soluble in acids and is remarkably characterized by the fixed acids including formic acid.

Originally, formic acid is a matter which is relatively unstable and easy to diffuse. In the method of the present invention, however, calcium ion which was instantly dissociated in a great deal by the reaction as mentioned above is quickly connected to formic acid for fixing this as formic acid calcium. Lower fatty acids such as formic acid are originally contained in the putrefactive waste but can be produced through thermal decompositions of protein or amino acid in the putrefactive waste by said reaction heat. Thus newly produced lower fatty acids including formic acid are also fixed as calcium salt utilizing said calcium ion. However, fixation of formic acid i.e., formation of calcium salt base is unduly dif-

4

ficult, in case of using calcium hydroxide as the additive. In this case, formic acid is defused into air or flown away by rain water etc.,.

Through the chemical reaction, higher fatty acids contained in the putrefactive waste are also formed as calcium salt in a similar manner.

Of any other organic matters contained in the putrefactive waste, e.g., water-soluble phosphate acid has about 98 % thereof fixed as phosphate acid calcium in effective form, which is produced through a reaction between phosphate acid contained in the putrefactive waste, phosphate acid contained in phospholipid and the additive. This results in a significant reduction in the water-soluble phosphate acid and lipid which have been contained in the putrefactive waste, the raw material.

In case of the putrefactive waste containing a relatively large amount of glyceride, this glyceride is converted to hardly dissolved calcium ion in stabilized status through an utilization of calcium oxide with highly active energy. This keeps the reaction product itself from producing anaerobic fermentation or gas or allowing harmful insects to grow.

Remainders of calcium ion not participating in the production of calcium ion, some of which are converted through a slaking reaction to calcium hydroxide and the other are converted to calcium carbonate through a contact with carbonic acid gas. Both exist in mixed status in the reaction product. As one of examples of proportion of calcium compound including calcium salt, there is a constitution of about 18% calcium hydroxide, about 48% calcium carbonate and about 34 % calcium salt, based on the amount of the added calcium oxide of 100 %.

By addition of calcium oxide, calcium compound is produced. Another effect by the addition is gasification and then removal of basic components such as ammonia or amine contained in the putrefactive waste. In other words, the addition of calcium oxide effects denitrification at a time of the hydration reaction that will allow adjustment of the nitrogen content in the reaction product to the appropriate. Therefore, phenomenon of the over-richness of nitrogen, deterioration in the product quality by post-fermentations and formation of anaerobic environment are prevented in the culture soil which was produced by fermenting the present product with aid of the microorganisms as will be mentioned surviving in the product.

The reaction time with the additive, if excessively long, presents kneading phenomenon (forming the treated into one in paste or into the fine-grained). The culture soil to be produced is, therefore, hardly formed in a structure wherein each of soil-grains getting together grow to a larger mass and hardly dried. For this reason, generally advantageous is within 15 minutes. The reaction time, however, is appropriately extended, where the raw material contains hardly reacting materials such as phospholipid, liquid oil, basic substances and hardly dissolved macromolecule compound.

Addition of the additive is not needed in all at once. In place, it may be added at times by divided into parts. Thus, the reaction product becomes a substance containing a mixture of un-reacting organic and in-organic matters and the above mentioned-calcium compounds which are, among others, physically uniformly dispersing into every direction (in every three-dimensional direction) by said activation of calcium oxide.

Next, explanations are made to the various properties and characteristics of the produced reaction product which are observed from various aspects, in conjunction with tables. At first, tables 5 to 7 show the results of the investigation into the respective working-manners and characteristics as the fertilizers of nitrogen, phosphate acid and potassium in the present reaction product. Table 5 shows that nitrogen compound was low in the rate of the amount of the in-organized in comparison with the control, the rate of the decomposition is slow and thus has delayed effectiveness as a fertilizer. Table 6 shows that general whole amount of phosphate acid is of citric solubility and thus has delayed effectiveness as a fertilizer similarly. Table 7 shows that more than 80% of potassium is of water-solubility and thus works quickly as a fertilizer.

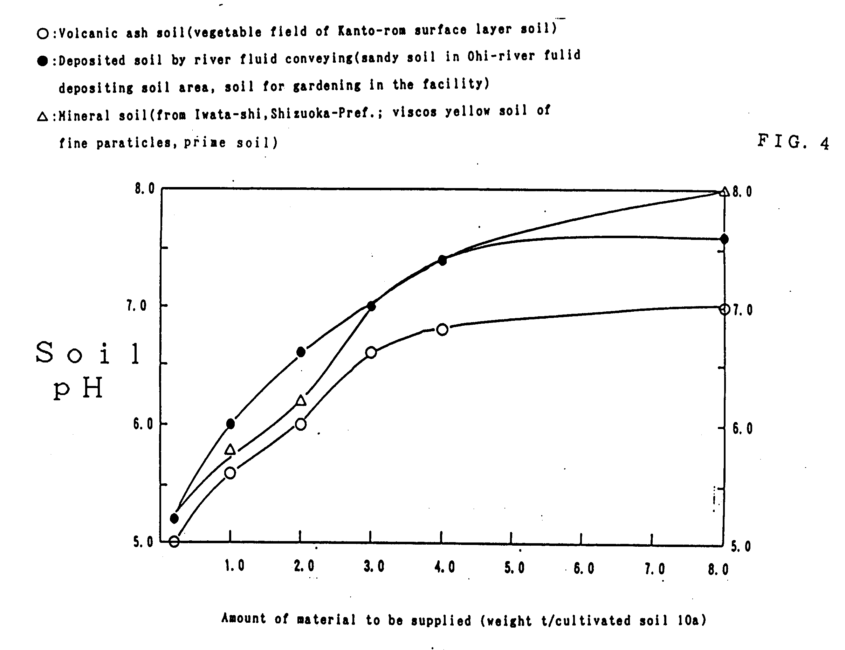

Table 8 shows the distributive rates of the molecular form of calcium compound in the reaction product according to the present invention. As is evident from this, calcium salt compound of 34 % exists in the reaction product. Further, Fig. 4 shows the curve of buffering pH in the soil when supplying the reaction product for the soil. In associated with the calcium proportion in the table 8, the illustrated in Fig. 4 shows that this reaction product is a substance which is alkaline and raises pH value.

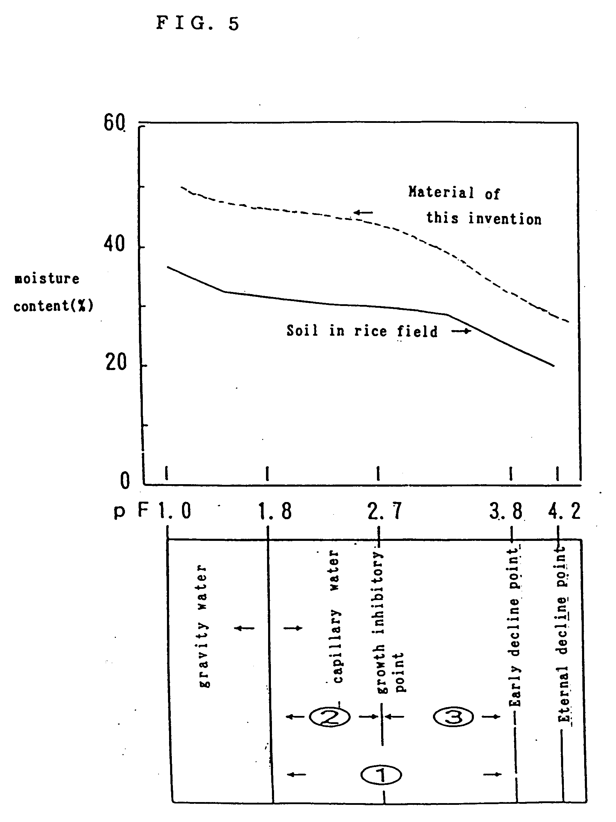

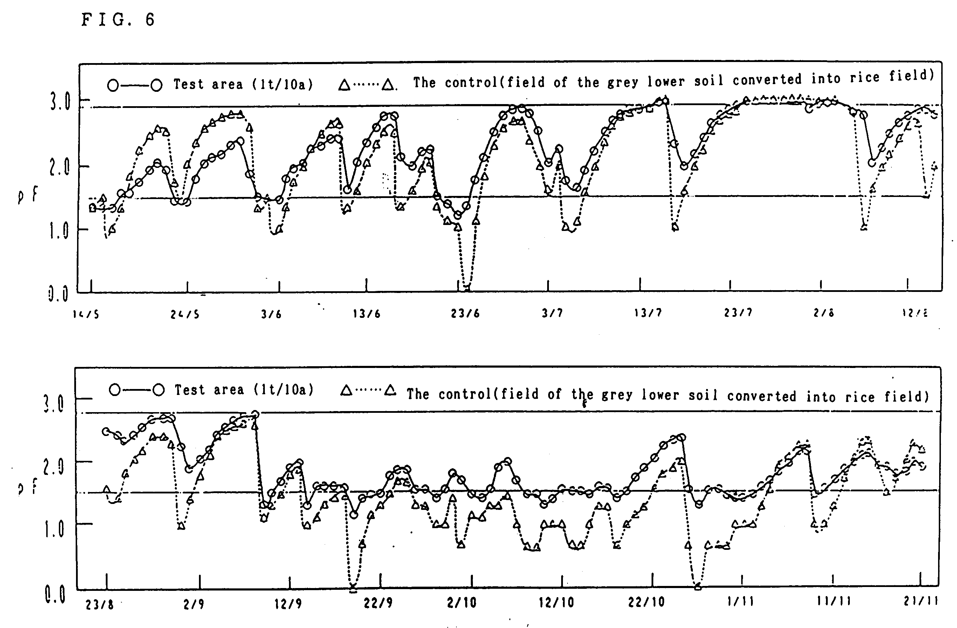

Table 9 shows the result of the investigation into the physical characteristics of the present reaction product. As is also shown on the water curve in Fig. 5, the present reaction product is found to be excellent in the ability of the water retention. In the Fig. 5, ① indicates an available water area of easily moving, ② indicates an available water area for normal growth and ③ i ndicates an available water area for abnormal growth. Relative to three-phases distributions, the table 9 also shows that there was a decrease in the rate of the solid phase while that of liquid and vapor-phase, each increased in comparison with each of the controls. Moreover, co-efficient of the water permeability into soil indicated a value about three times as high as that of the control. Over the water areas of easily and hardly moving in the test area, pF indicated that there was an increase by about 40 % in the amount of the contained water. See Fig.6 suggesting movements of the water where water is supplied into the soil for further evidence. Fig. 6 shows that pF in the control decreased to 0 during a period of no-rain but showed the extremely rising curve at a rainy time. In contrast, pF in the area supplied with the present reaction product is maintained at as much as 2.5 in average. This data suggests an improvement in the ability of the water retention.

The reaction product having such physical and chemical properties is sterilized by the co-actions of the hydration reaction heat and the highly alkalinity in the process of being treated thereby providing an environment wherein specific microorganisms such as alkalophile microorganisms only live. After treatment of the reaction product, the organic acids which are to be utilized as carbon sources of the microorganisms are converted to hardly dissolved calcium salt through the reaction with calcium oxide. Further, the reaction product is under highly alkaline environment with pH of 12 or more.

As was shown in Fig. 1, the reaction heat in the process of the reaction instantaneously vehemently have risen. In addition, the reaction product as the resultant is in highly alkaline status as mentioned above. This forces, most of the microorganisms which have been contained in the putrefactive waste in the process of the reaction product being treated, into the sterilization, except for microorganisms only which survived from this environmental change.

In case of the reaction product which has originally been treated from the swine excreta, these microorganisms as survivors include bacteria, filamentous fungi and 90-A1 bacterium (assumed to be actinomycete) as shown in table 10 to 13 as will be mentioned.

The bacteria was a dominant species detectable at a level of e.g., 104/g. Filamentous fungi and actinomycete were too few to be detect even at a level of 102/g. The bacteria isolated here, all formed heatstable spores and aerobically grow. Therefore, most are in the genus Bacillus. These microorganisms, every is in dormant status in the reaction product.

The table 13 shows the growth condition of the bacteria in dormant status.

Among these microorganisms, some were identified as new species, the ground of which is follows.

Strain No. GM-4 (this strain corresponds to No. 0-7, a representative strain in the table 10 or 13 and to No. FERM P-13578 as the deposited to Patent Microorganism Deposit Center [Japan Micro Deposit Center] - N.B. All Deposit N$^{os}$ listed in this specification relate to this center) formed oval to long oval spores and showed filamentous colony on the agar medium. The optimum pH for its growth was in the range of 7 to 11. As the physiological character, this strain showed catalase positive, VP reaction negative, MR reaction negative and the decomposition of starch negative. This strain was therefore, assumed to be similar to a strain of Bacillus badius. This strain was, however, different from B. badius in that this strain could reduce nitrate to nitrite and utilize citric acid. These data strongly suggested that this strain should be identified as a strain of new species in the genus Bacillus.

Strain No. GM-5 (this strain corresponds to No. 30-2, a representative strain in the table 10 to 13 and to No. FERM P-13579 as the deposited to Patent Microorganism Deposit Center) was a bacterium of aerobic gram positive rod and could form spores. This strain showed catalase positive, VP reaction negative, MR reaction negative and the decomposition of starch negative. Therefore, this strain was assumed to be similar to a strain of B. brevis, B.sphaericus or B.pasteurii. This strain was different from the B.brevis in that this strain showed urease positive, negative in abilities of decomposing gelatin and casein, grew on the agar medium containing 7% NaCl and produced an acid utilizing Xylose. Further, this strain was different from B.sphaericus in that this strain formed oval spores, not spherical, had ability of reducing nitrate to nitrate and produced acids utilizing Xylose and D-Mannitol. Furthermore, this strain was different from B.pasteurii in that this strain formed oval spores, failed to grow under the anaerobic condition and failed to decompose gelatin. Those data strongly suggested that this strain should be identified as a strain of a new species in the genus Bacillus.

Strain No. GM-8 (Deposit No. FERM P-13581) was a bacterium of aerobic, gram positive rod and could form spores. This strain showed catalase positive, VP reaction negative, MR reaction negative and the decomposition of starch positive. Therefore, this strain was assumed to be similar to B. firmus. This strain, was, however, different from B. firmus in that this strain could grow at 55 °C and had ability of utilizing citric acid and failed to produce an acid utilizing mannitol. These data strongly suggested that this strain should be identified as a strain of a new species in the genus Bacillus.

Strain No. GM-9 (Deposit No. FERM P-13582) was different from B.subtilis in that this strain formed some spherical spores, was negative in the ability of reducing nitrate to nitrate and failed to produce acids unitizing arabinose, Xylose and D-mannitol and demanded the growth factor.

Strain No. 90-4 (Deposit No. FERM P-14218) was different from B.lentus in that this strain formed spherical spores and could grow at 50 °C.

Strain No. 90-7 (Deposit No. FERM P-14219) was different from B.brevis in that this strain showed urease positive, failed to decompose starch and could grow on the agar medium containing 7 % NaCl. This strain was also different from B.sphaericus in that this strain formed short oval spores and was positive in the ability of reducing nitrate to nitrate. Furthermore, this strain was different from B.pasteurii in that this strain formed short oval spores and failed to grow anaerobically,.

Strain No. 90-11 (Deposit No. FERM P-14213) was different from B.brevis in that this strain formed no-swollen spores, showed urease positive and failed to decompose casein and could grow on the agar medium containing 7 % NaCl. Also, this strain was different from B. sphaericus in that this strain formed oval spores without swollen sporangium and was positive in the ability of reducing nitrate to nitrite. Moreover, this strain was different from B.pasteurii in that this strain produced oval spores without swollen sporangium and failed to grow anaerobically.

Strain No. 90-1 (Deposit No. FERM P-14215) was different from B. firmus in that this strain grew at 55 °C, formed swollen sporangium, could grow anaerobically and utilized citrate acid and failed to produce acid utilizing mannitol.

Strain No. 90-8 (Deposit No. FERM P-14220) was different from B.firmus in that this strain showed negative in the ability of casein hydrolysis, negative in the production of acid utilizing glucose and D-mannitol and could grow at 55 °C.

Strain No. 90-2 (Deposit No. FERM P-14216) was assumed to belong to Coryneform bacteria. This strain was, however, different from Coryneform bacteria in that this strain contained ornithine as the cell wall diamino acid and MK-8 (H4) as the Quinone. Bergey's Manual introduced no microorganisms which contain Ornithine as the cell wall diamino acid and is ornithine and MK-8(H4) as the Quinone. Therefore, this strain was assumed to be identified as a strain of the new genus.

Strain No. 90-3 (Deposit No. FERM P-14217) has a possibility of identified as bacteria in the new genus because this strain was different from the bacteria in the genus Cellulomonas in that this strain failed to decompose cellulose.

Strain No. 90-10 (Deposit No. FERM P-14221) has a possibility of identified as the bacteria in the new genus because this strain was different from the bacteria in the genus Cellulomonas in that this strain failed to decompose cellulose.

Relative to the strain No. 90-3 and 90-10, there are differences therebetween in the abilities of casein decomposition, the growth on the agar medium containing 7 % NaCl, the utilization of propionate and the starch hydrolysis. These strains are, nevertheless, assumed to be ones in the bacteria very similar to each other.

Strain No. 90-F1 (Deposit No. FERM P-14214) was characterized by having large spherical conidia. Formation of the conidia was assumed to be in type of phialoconidium, which was different from typical Penicillium type. Furthermore, this strain have many characteristics to be investigated.

Strain No. 90-A1 (Deposit No. FERM P-14222) failed to forme its mycelium into fragmentation and thus failed to form aerial mycelium. Diamino pimelic acid under the hydrolysis of the mycelia was meso-DAP. This strain grew well on the yeast extract, malt extract agar medium and formed colony. The characteristics of the colony was the point of the coral red and butter-like colony.

Representatively, included in the bacteria are the strain No. GM-4 and GM-5 (GM-4 corresponds to No. 0-7, the representative strain in the table 10 or 13 and No. FERM P-13578, as the deposited to Patent Microorganism Deposit Center / GM-5 corresponds to No. 30-2, the representative strain in the table 10 or 13 and No. FERM P-13579, as the deposited to Patent Microorganism Deposit Center). GM-4 was found to be the similar to B .badium but was different from B.badium in that this strain could utilize citric acid and grow at pH 11. GM-5 was found to be similar to B.sphaericus but was different from B. sphaericus in that this strain produced acid in subtle utilizing glucose and failed to grow at pH of 6.8.

Further, the bacteria included the strain No. GM-8 and GM-9 (GM-8 corresponds to strain No. 30-1 in the table 10 or 13 and to No. FERM P-13581, as the deposited to Patent Microorganism Deposit Center / GM-9 corresponds to strain No. 30-1 in the table 10 or 13 and to No. FERM P-13582, as the deposited to Patent Microorganism Deposit Center).

As is mentioned above, the microorganisms in the reaction product are in dormant status. Therefore, decomposition of the organic matters with aids of the microorganisms is not proceeded in the reaction product with the putrefaction suspended. For this reason, the putrefaction of the reaction product is not progressed although there is slightly sensible stint (of the excrement) given off by the microorganisms.

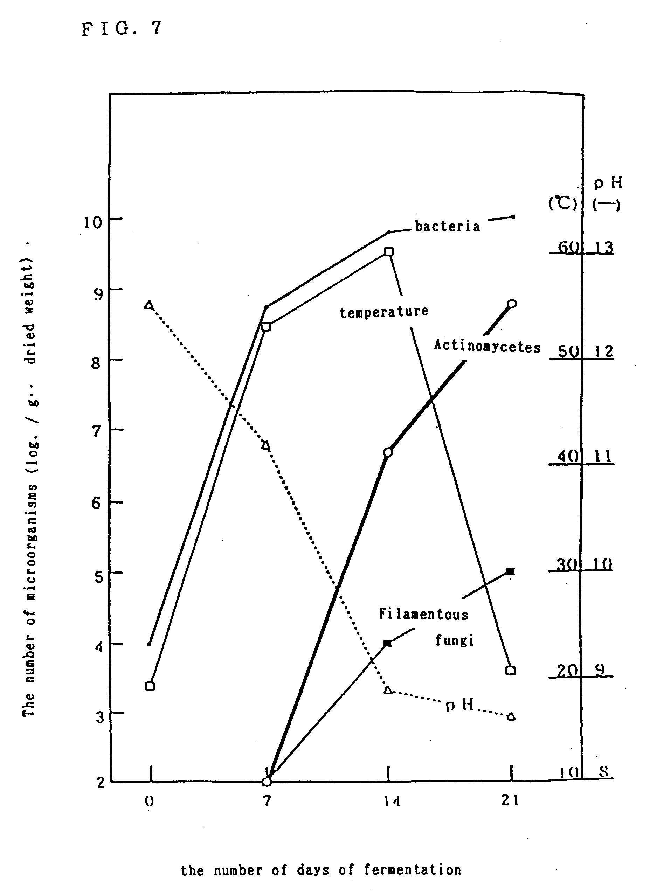

Where the slaked lime was mixed into the putrefactive waste, high reaction heat can not be obtained. Therefore, the rate of the lower fatty acid representatively including formic acid converted to calcium salt is low and the denitrification of ammonia etc., does not occur. Another effect by this mixing is mere aggregation of un-decomposed solid matters around the slaked lime which fails to assist in uniformity in pH of the composts. Still, the reaction product contains a large amount of the water soluble components and general microorganisms surviving in a considerably great deal. The proliferation of the microorganisms producing acids such as ones in Enterobacteriaceae, therefore, is permitted, causing the declination in pH of the present reaction product which was rated at 12 or more just after the production. The pH decline, in return, permits an instantaneous proliferation of the any other microorganisms restricted so far, which is followed by the outbreak of an abnormal fermentation together with generation of the stink. In contrast, the reaction product according to the method of the present invention, for the reason as mentioned above, is freed from troubles caused by the un-decomposed organic matters such as the generation of the harmful gas or the proliferation of the harmful microorganisms. In supplying water and air at the desired period, this reaction product is neutralized by utilizing carbon dioxide in the air to have its pH decreasing to 12 or less. This stimulates those bacteria surviving under the alkaline environment to start each proliferation (see Fig.7). Especially, the strain No. GM-4 can proliferate under the highly alkaline environment (with pH 11 to less than 12)(see table 13). Therefore this strain dominantly starts its proliferation.

Along with this fermentation, pH is lowered. With the decline in pH, the proliferation of any other bacteria restricted so far is permitted. When the proliferation of the strain GM-4 is progressed to some extent, the strain No. GM-5 is encouraged to starts its proliferation because this strain can proliferate under the environment with pH of 10.

Along with the proliferation of the both GM strains, the reaction heat is generated with the temperature of the reaction product rising. This stimulates the strain No. GM-8 (No.FERM P-13581, the deposited to Patent Microorganisms Deposit Center) and GM-9 (No.FERM P-13582, the deposited to Patent Microorganisms Deposit Center which has been in dormant status to start each proliferation. The strain No. GM-9 is the similar to B. subtilis well-known as one generating heat as will be mentioned. Further increase in the temperature of the reaction product is therefore provided.

With the proliferation of these bacteria increasingly activated, further decline in pH is promoted in the reaction product. When this decline in pH reaches a level at which actinomycete and filamentous fungi can proliferate, these bacteria whose number were initially too few to be detected start each proliferation. Both are the microorganisms having durable spores because they are survivors under said high-temperature and highly alkaline environment.

Said strain No. GM-8 was turned out to be one similar to B.firmus but was different from B. firmus in that this strain could utilize citric acid and grow at 50°C. The strain No. GM-9 was turned out to be similar to B.substilis but was different from B.subtilis in that this strain failed to grow at pH of 5.7 and could utilize propionic acid.

When the increase in the number of the Bacillus reaches the stationary phase, the proliferation almost stops with the room temperature decreasing. This stimulates the proliferation of the actinomycete and filamentous fungi even more. Therefore the soluble organic matters in the reaction product are utilized by these microorganisms. At the same time, metabolites from the bacteria are accumulated in the reaction product. Further, calcium hydroxide in the reaction product is converted to calcium carbonate through sequential fermentative operations by the microorganisms.

As a result, the reaction product is converted to a fermentative product having the physical and chemical properties effective for plants with aids of the microorganisms.

EMBODIMENTS

Hereinbelow, embodiments of the present invention will be shown as follow.

Highly activated calcium oxide of 10 Kg was added to the swine excreta of 100 Kg as a raw material. Both were mixed and stirred up for 15 minutes in a reaction machine whereby a reaction product of 110 Kg in slurry status was obtained. Thus obtained product was dried in the drying chamber having temperature of 30°C for removal of about 20 % water. Then the product was in custody. After 60 days, it was introduced into a fermentation pool and picked out after about 72 hours whereby a fermentative product of about 40 Kg was obtained. Thus obtained fermentative product comprises calcium compounds of 15 Kg (in dried weight) and fermented organic matters and any other in-organic matters of 12 Kg (in dried weight).

Relative to pH value or the like and weight % of calcium, magnesium and kalium in the composite component of the fermentative product, comparisons are made with those of the reaction product. At first, the reaction product was of 12.6 in pH, 6.9 in EC, 28.2 % in the total amount of calcium, 1.54 % in the total amount of magnesium and 1.48 % in the total amount of potassium. On the other hand, the fermentative product was of 7.78 in pH, 2.62 in EC, 25.8 % in the total amount of calcium, 0.88 % in the total amount of magnesium and 0.66 % in the total amount of potassium.

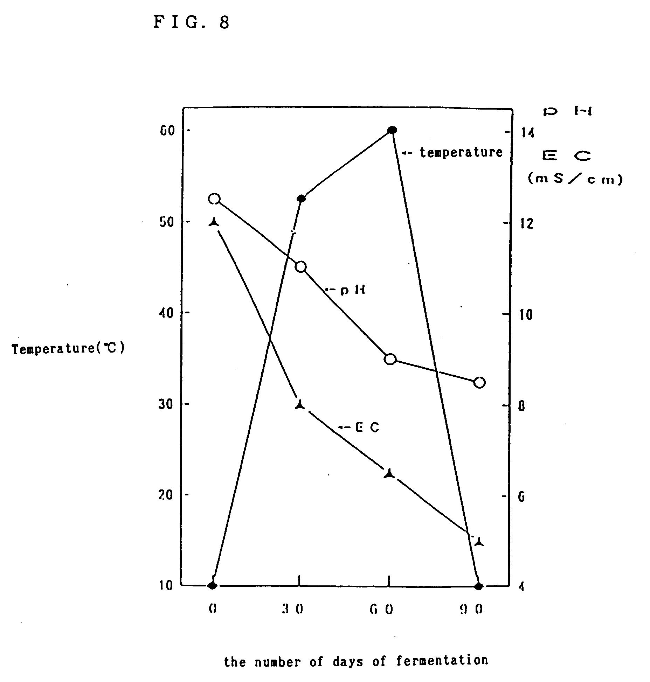

Fig.8 shows changes in pH and EC with the passing of time when the reaction product obtained through said procedures was supplied with the adequate water and fermented thereby. As is evident from Fig. 8, there was a vigorous rising of the temperature for first 7 days and then the gradual rising until 14 th days, i.e., for following 7 days. Thereafter, a vehement and instantaneous declination occurred and the initial room temperature was returned after 21 days. For 14 days during which the rising of temperature was observed, pH declined linearly from 12, 13 to around 8.6. After the 14 th day at which the declination in the room-temperature started, the linear declination was gently angled and showed almost a horizontal line. On the other hand, EC violently declined from 12 to 8 for first 7 days and then showed another linear declination from 8 to 5 at a slightly gentle angle for following 14 days.

Fig.7 shows variances of the microorganisms in the process of the fermentation shown in Fig.3. Of those microorganisms, first proliferating were bacteria. Actinomycete and filamentous fungi were not detected at this stage. A sudden increase of the bacteria occurred at several days after the fermentation started. The proliferation period is roughly divided into about 20 days consisting of the first and the middle stages of logarithmic phase and following about 60 days as the late stage of the logarithmic phase showing a curve of the growth. When pH declined to 9 or less at 60 th days from the start of the fermentation, actinomycete and filamentous fungi was detected finally. In this late stage, a remarkable growth of actinomycete was found, in especial.

As mentioned above, the present reaction product is a fermentative substance which provides the environment preventing the growth of the bacteria in the genus Enterobacteriaceae and common bacteria. The fermentation of the present reaction product was, therefore, strongly suggested to be carried out by the specific microorganisms adaptable to the present environment. These microorganisms are, however, not involved in the entire period of the fermentation. It is interpreted that various microorganisms can adapt to variable environments with the passing of time and each appear as the dominant one to assist in the fermentation.

The fermentative product was characterized as follows;

Color:      Brown to weakly blackish brown.
Smell:      Compost smell or hardly sensible.
Shape:      Unrecognizable as the original shape. Small grain-shape.

Water content:

Hardly attached on the palm even if firmly grasped.
Water retention rate of about 30%.

In the standard for determining degrees in ripeness (Harada; 1984), the product can be graded in the supreme class.

This fermentative product was directly able to be used as the compost or the culture medium satisfying maturity conditions. Especially, it was generally able to be used as the culture soil for raising various crops such as melon, komatsuna (a kind of Chinese cabbage), spinach, eggplant, tomato, cucumber. Where the fermentative product is employed as the culture soil for these crops, various organic acids contained in calcium salt are in form of easy to be absorbed into roots of culture plants and act in attempt to enhance properties of the respective. Examples of the enhancement by the acids are an improvement in sugar content, a declination in the rate of the water content, an improvement in resistance to diseases, an increase in the amount of contained starch and an improvement in leaf thickness. Another example of the enhancements as reported includes a blossom period made early, improved deep roots spreading or the like.

From September to December in 1993, cultivation tests on melon were carried out in Iwata agriculture high school in Shizuoka prefecture. In the test, a humus rich rice field were used as the control area while an area supplied with the fermentative product, a resultant from the method of the present invention was used as the test area. Supplied fertilizer was an organic-blending N component exclusively used for melon. 4 times supplies was made at every 10 g per strain. In the test area, supplemental fertilizing of calcium oxide and potassium was not given. Advantageously, the testing area was of 14.5 % in the rate of the sugar content in the fruits with about 1 % rising suggested. On the other hand, 13.6% was indicated in the control.

Another advantage of the present fermentative product is found in having preferable effects on the growth of grains, eatable roots, leaf vegetables or the like in the rice field and yard, when supplied for cultivating therefor.

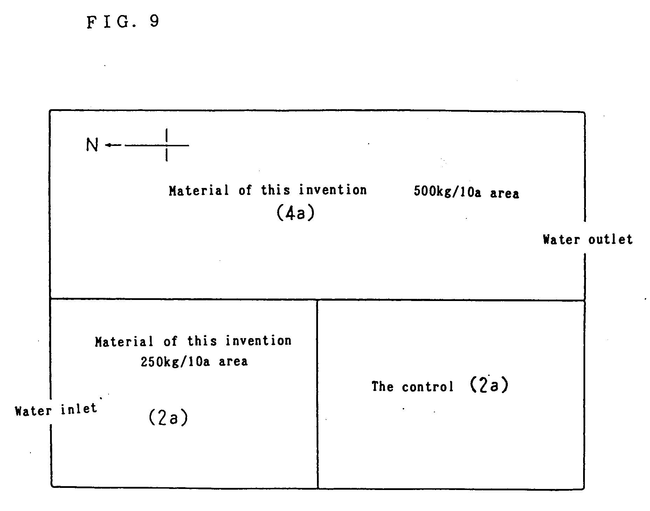

From 1991 to 1992, cultivation tests on rice were carried out in the rice field under the conditions as shown in the table 14. The test results were obtained as is shown in table 15 relative to the amount of the harvest and also as is shown in table 16 relative to the quality and taste. Fig. 9 shows the scale and the arranged compartment of the testing area. It was found that production of those excellent in taste was advantageously achieved in the testing area, although the amount of the harvest was almost equal to that of the control. Table 17 shows compared values of the components contained in the soil previously used for the rice field. The comparison reveals that there was no accumulation of calcium and no increase in soil pH although the soil was a substance utilizing calcium.

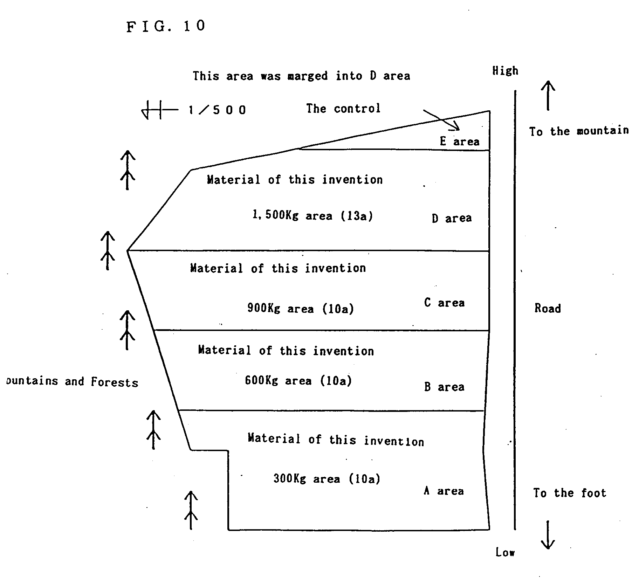

From 1991 to 1992, cultivation tests on barley were carried out using the present fermentative product at the scale and the arranged compartments as shown in Fig. 10 under the cultivation condition as shown in table 18 as will be mentioned. The tests results were obtained as shown in tables 19 and 20. The area E which had been the control without supply of the present substance in the first year was merged into the area D in the second year, resulting in elimination of the control in the second year. In place, the cultivation area of the area D increased to 14a. Table 20 shows a significant increase in the amount of the harvest in each of the first and second years.

In 1991, cultivation tests on sweet potato were carried out using the present fermentative product under the cultivation condition as shown in table 21. The tests result was obtained as shown in table 22 as will be mentioned, which revealed that there were increases by 30 % not only in the substantial amount of the harvest but also average weight per piece. The table 22 also shows an increase in the amount of starch and carbohydrate.

From 1990 to 1991, cultivation tests on yam were carried out using the present fermentative product under the cultivation conditions as shown in table 23 to 28 as will be mentioned (table 23 shows composition of the garden for exhibition. Table 24 shows content of the testing area. Table 25 shows outline of seeds. Table 26 shows the amount of the fermentative product to be supplied. Table 27 shows the using number of chemicals. Table 28 shows the weather condition). The tests result was obtained as shown in table 29 and 30. The table 29 as will be mentioned shows the comparison of the quality standards and reveals that those uniform in the quality standards was obtained even in the third year. The table 30 shows the comparison of the qualities such as the amount of starch and reveals that the quality of the harvest in the testing area was superior to that of the control in the first and third years.

In 1989, cultivation tests on soy bean were carried out using the present fermentative product under the cultivation conditions in table 31 as will be mentioned. The tests result was obtained as shown in table 32. The table 32 shows the comparison of the amount of the pods as the harvest with two or three beans and reveals that the obtained was those in totally average in the amount of the harvest and was at least equal to that obtained from the control.

In 1988, sequential cultivation tests on spinach were carried out using the present fermentative product under the cultivation conditions as shown in table 33 as will be mentioned. The test results are obtained as shown in table 34, which shows remarkable effects of the present product on the growth and the amount of the harvest.

The effect by sequential use of the present fermentative product on the formation of the soy bean root nodule was investigated under the cultivation conditions as shown in table 35. The result was obtained as shown in table 36, which reveals that there was an increase in the weight of the root nodule by about 40 % in average, in comparison with that of the control.

The effect by sequential use of the present fermentation material on the root disease of yam was investigated. The result was obtained as shown in table 37. In this table, 0 indicates no-occurrence of the disease and number 1 to 4 indicate the degree of the disease. Larger number shows more serious symptom. In the control in the second year, the present fermentative substance was used. This year is, therefore, regarded as a year when the present fermentative substance was solely used.

According to the table 37, it was found that the degree of the appearance of the disease was significantly low where the present fermentative substance was used.

Table 1

| Item / Sample | | CaO Addition Rate (%) | Ca (%) | Organism Amount (%) | Calsium Organism Ratio (%) | All carbons (%) | | |
|---|---|---|---|---|---|---|---|---|
| | | | | | | Measure--ment value | Conver--sion value | Decrease rates |
| Control thing | | 0.0 | 4.2 | 77.7 | ----- | 45.4 | ----- | ---- |
| CaO dition process--ing thing | F5 | 5.0 | 20.3 | 51.4 | 0.39 | 29.8 | 45.5 | < 1 |
| | F10 | 10.0 | 29.8 | 37.1 | 0.81 | 21.5 | 45.0 | < 1 |
| | F20 | 20.0 | 37.8 | 26.5 | 1.41 | 15.4 | 45.2 | < 1 |

table 2

| Item / \ple | | CaO Addition Rate (%) | $NH_4$-N(mg/Kg) | | | $NO_3$-N | All nitrogen (%) | | |
|---|---|---|---|---|---|---|---|---|---|
| | | | Measure--ment value mg/Kg | Conver--sion value | Decrease Rate | Measure--ment value mg/Kg | Measure--ment value | Convers--ion value | Decrease Rate |
| Control thing | | 0.0 | 38,800 | ---- | ---- | < 50 | 7.43 | ---- | ----- |
| CaO addition process--ing thing | F5 | 5.0 | 207 | 313 | 99.2 | < 50 | 2.39 | 3.64 | 51.0 |
| | F10 | 10.0 | 150 | 314 | 99.2 | < 50 | 1.74 | 3.63 | 51.0 |
| | F20 | 20.0 | 120 | 352 | 99.1 | < 50 | 1.26 | 3.69 | 50.3 |

Table 3

| Item / Sample | | Lipid (mg/Kg) | | | Oil (mg/Kg) | | | Acetic acid (mg/Kg) | | | Butyric (mg/Kg) | | |
|---|---|---|---|---|---|---|---|---|---|---|---|---|---|
| | | Measurement value | Conversion value | Decrease Rate | Measurement value | Conversion value | Decrease Rate | Measurement value | Conversion value | Decrease Rate | Measurement value | Conversion value | Decrease Rate |
| Control thing | | 81,300 | ------ | ---- | 65,300 | ------ | ---- | 47,600 | ------ | ---- | 15,300 | ------ | ---- |
| CaO addition processing thing | F5 | 18,500 | 27,966 | 65.6 | 10,300 | 15,570 | 76.2 | NT | ------ | ---- | NT | ------ | ---- |
| | F10 | 8,760 | 18,346 | 77.4 | 6,230 | 13,048 | 80.0 | 40,700 | 85,240 | -79. | 12,800 | 26,807 | -75. |
| | F20 | 7,320 | 21,462 | 73.6 | 4,830 | 14,162 | 78.3 | NT | ------ | ---- | NT | NT | ---- |

table 4

| Item / Sample | | $P_2O_5$ | | | | | | MgO (%) | | | $K_2O$ (%) | | |
|---|---|---|---|---|---|---|---|---|---|---|---|---|---|
| | | Water soluble phosphorus asid (mg/kg) | | | All phosphorus asid (%) | | | | | | | | |
| | | Measurement value | Conversion | Decrease Rate | Measurement value | Conversion | Decrease Rate | Measurement value | Conversion value | Decrease Rate | Measurement value | Conversion value | Decrease Rate |
| Control thing | | 15,900 | ---- | ---- | 5.54 | ---- | ---- | 1.94 | ------ | ------ | 5.18 | ---- | ---- |
| CaO Addition Processing thing | F5 | 148 | 224 | 98.6 | 2.93 | 4.23 | 14.4 | 1.31 | 1.98 | -2.1 | 2.58 | 3.90 | 24.7 |
| | F10 | 27 | 56 | 99.6 | 1.86 | 3.90 | 28.4 | 1.43 | 2.99 | -54.1 | 2.13 | 4.46 | 13.9 |
| | F20 | 11 | 32 | 99.8 | 1.38 | 4.05 | 25.6 | 1.49 | 4.96 | -155.7 | 1.51 | 4.43 | 14.5 |

Table 5

| Culture period / Amount of making of materials inoragic | | 30° C culture period (day) in state of field | | | |
|---|---|---|---|---|---|
| | | 2 | 7 | 14 | 4 |
| Con--trol Mate--rials (ripe com--post) | Amount of making to inorganic (mg/100g drysoil) | 1.47 | 3.66 | 4.98 | 6.37 |
| | Rate of making to inorganic (%) | 2.9 | 7.3 | 10.0 | 12.7 |
| Mate--rial of this inven--tion | Amount of making to inorganic (mg/100g drysoil) | 1.83 | 2.76 | 3.64 | 5.85 |
| | Rate of making to inorganic (%) | 3.7 | 5.5 | 7.3 | 11.7 |

Table 6

| Dissolusion form / Amount of dissolution | Whole quantitiy = 2.26% | | |
|---|---|---|---|
| | Water solublu | Solubili -ty | Acid solublu |
| P₂O₅ (groceries%) | 0.06 | 1.54 | 2.15 |
| Dissolve rast (whole quantity%) | 3 | 68 | 95 |

table 7

| Dissolution form / Amount of dissolution | Whole quantity | Water solublu |
|---|---|---|
| K₂O (groceries %) | 1.48 | 1.23 |
| Dissoive rate (whole quantity %) | ------- | 83 |

Table 8

| Item | Analysis result | | |
|---|---|---|---|
| Amount of Ca in materials of this invlntion | 29.8%(a) | | |
| Analysis method | Curve of neutralizing of ⟨suitablu⟩ | Weight method | (a)-(b+c) |
| Analysis value: Ca% | 5.3 (b) | 14.2 (c) | 10.3 |
| Quality for analysis | Ca(OH)₂ | CaCO₃ | Calsium chioridization combination thing |
| | | | |
| Distribution mixing by which CA in materials is assumed to be100 | 18% | 48% | 34% |

Table 9:()is a comparison rate to assume the contrast soil to be 100.

| Sample \ Item | Three aspect distribution | | | Coefficient of water permeability soil | | Effective content of fective water retention ml/50mlV | Easiby moving water ml/50mlV | Hordy moving water ml/50mlV |
|---|---|---|---|---|---|---|---|---|
| | S | L | G | | | | | |
| Control soil (rice field soil ) | 30 | 22 | 48 | 3.0 | 6 (100 ) | 6.82 (100) | 1.92 (100) | 4.90 (100) |
| This manufactur- -ing Materials of this invention | 17 | 31 | 53 | 8.9 | 6 (293 ) | 9.56 (140) | 2.81 (146) | 6.75 (138) |
| Note | The unit is %. | | | Orde $\times 10^{-3}$ | r | pf1.8 ~3.8 | pf1.8 ~2.7 | pf2.7 ~3.8 |

Table 10

| Bacillus group | Number of bacilli | Fermention period (×106/gm \<dryness\> amount) | | | |
|---|---|---|---|---|---|
| | | Before fermenting | The fermentation first term | Fermentation middle term | Fermentation latter term |
| Bacillus group | 1 | 0.0063 | | | |
| | 2 | 0.0005 | | | |
| | 3 | 0.0017 | 73 | | |
| | 4 | 0.0008 | 159 | 975 | |
| | 5 | | 12 | | |
| | 6 | | | 81 | |
| | 7 | | | | 1,160 |
| | 8 | | | | 580 |
| | 9 | | 25 | 410 | |
| | 10 | | | | 1,160 |
| | 11 | | | | 1,160 |
| | 12 | | | 331 | |
| | 13 | | | 81 | 1,160 |
| | 14 | | | | 2,320 |
| | 15 | | | | 1,740 |
| | 16 | | | | 1,740 |
| | 17 | | | 81 | |
| Number of total bacilli | | $0.00933 \times 10^6$ | $269 \times 10^6$ | $1959 \times 10^6$ | $11020 \times 10^6$ |

Table 11

| Amount of microorganism / Microorganism group | Fermentation period | | | | Comparison microorganism kind |
|---|---|---|---|---|---|
| | Before fermenting | The fermenting first term | The fermentation Middle term | Fermentation latter term | |
| Bacillius | $0.00933 \times 10^6$ | $269 \times 10^6$ | $1.950 \times 10^6$ | $11.020 \times 10^6$ | Bacillus sp.Coryne form type |
| Radiation bacter ium | 100 less than | 100 less than | $3.31 \times 10^6$ | $339 \times 10^6$ | Rhodococcs rhodoch- -rous |
| Fulanentous Fungi | 100 less than | 100 less than | $0.0078 \times 10^6$ | $0.135 \times 10^6$ | Penisi- -llium sp |

Table 12

| Revitalization item / Fermentation Period | GROUP | Growing environment | | | Ferment revitalization | | | Use of organic acid | | |
|---|---|---|---|---|---|---|---|---|---|---|
| | | pH 6 | pH 10 | 50 °c | (*puroteah ze*) | (*ureahze*) | (*amirah ze*) | Acetic acid | (*kuen*) acid | (*kohak*)acid |
| Before fermention | 1 | + | - | - | - | - | ++ | - | - | - |
| | 2 | - | W | - | ++ | + | ++ | + | + | + |
| | 3 | - | ++ | - | ++ | ++ | - | ++ | ++ | ++ |
| | 4 | - | + | - | - | ++ | - | - | - | - |
| The fermentation first term | 3 | - | ++ | - | ++ | ++ | - | ++ | ++ | ++ |
| | 4 | - | + | - | - | ++ | - | - | - | - |
| | 5 | - | W | - | - | + | - | ++ | ++ | ++ |
| | 9 | + | + | ++ | ++ | - | + | W | + | - |
| Fermentation middle term | 4 | - | + | - | - | ++ | - | - | - | - |
| | 6 | - | + | - | - | - | - | - | - | - |
| | 9 | + | + | ++ | ++ | - | + | W | + | - |
| | 12 | + | - | ++ | ++ | - | + | W | W | - |
| | 13 | - | -/W | -/W | - | - | + | -/W | - | - |
| | 7 | - | - | - | - | - | + | - | - | - |
| Fermentation latter term | 7 | - | W | - | W | ++ | - | W | ++ | ++ |
| | 8 | - | + | - | - | ++ | - | W | ++ | ++ |
| | 10 | + | + | + | + | - | + | ++ | + | - |
| | 11 | - | + | + | + | - | + | ++ | - | - |
| | 13 | - | -/W | -/W | - | - | + | -/W | - | - |
| | 14 | + | + | W | + | - | + | ++ | - | + |
| | 15 | - | W | W | + | - | + | ++ | - | - |
| | 16 | - | W | W | + | - | + | + | - | - |

Table 13 The Environment growing an isoldated Micro org amisw

| GROUP | Representative bacterium stpck No. | Composition of stock | Salt tolerance 7%NaCl growing | grouing temperature(°c) 40 | 45 | 50 | 55 | 60 | growin g/pH pH5 | pH6 | pH7 | pH8 | pH9 | pH10 | pH11 |
|---|---|---|---|---|---|---|---|---|---|---|---|---|---|---|---|
| 1 | 0-1 | 15 | ++ | + | + | - | | | - | + | ++ | ++ | + | - | - |
| 2 | 0-5 | 1 | +++ | + | | - | | | - | - | +++ | +++ | +++ | W | - |
| 3 | 0-7 | 10 | +++ | + | + | - | | | - | - | +++ | +++ | +++ | +++ | W |
| 4 | 30-2 | 27 | ++ | + | - | - | | | - | - | d | ++ | + | d | - |
| 5 | 30-5 | 1 | ++ | + | - | - | | | - | - | ++ | ++ | + | W | - |
| 6 | 60-13 | 1 | ++ | + | | - | | | - | - | ++ | ++ | +++ | + | - |
| 7 | 90-7 | 2 | + | + | - | - | - | | - | - | +++ | +++ | + | W | - |
| 8 | 90-11 | 1 | + | + | - | - | - | | - | - | +++ | +++ | ++ | + | - |
| 9 | 30-1 | 7 | ++ | ++ | + | ++ | + | - | - | W | +++ | +++ | ++ | + | - |
| 10 | 90-1 | 2 | +++ | + | + | + | + | - | - | + | +++ | +++ | ++ | + | - |
| 11 | 90-8 | 2 | + | + | + | + | - | | - | - | +++ | +++ | ++ | + | - |
| 12 | 60-7 | 4 | ++ | ++ | + | ++ | + | - | - | W | +++ | +++ | + | - | - |
| 13 | 90-4 | 2 | + | + | + | W | - | | - | - | ++ | ++ | + | W | - |
| 13 | (60-5) | 1 | + | + | | - | | | - | - | ++ | ++ | W | - | - |
| 14 | 90-2 | 4 | ++ | + | + | W | - | | - | + | +++ | +++ | ++ | + | - |
| 15 | 90-3 | 2 | - | + | + | W | - | | - | - | +++ | +++ | + | W | - |
| 15 | (90-9) | 1 | - | + | + | W | - | | - | - | +++ | +++ | + | W | - |
| 16 | 90-10 | 3 | W | + | + | W | - | | - | - | +++ | +++ | + | W | - |
| 17 | 60-20 | 1 | +++ | + | - | - | | | - | - | ++ | ++ | + | - | - |

Table 14

| Test place | Iwate Pref.Mizusawa-city flower garden |
|---|---|
| Test organization | It is a scene in the Iwate Pref. agriculture chemicals examination scene prefectu south. |
| Soil | ぼく of humidity black |
| The Control | materials,farmer,habitual practice,cultivation |
| Test akea | This materials 250Kg/10a districts and 500 Kg/10a districts |

| Outline of <cu vation> | Year first | Year second |
|---|---|---|
| Kind Style of growth bud Transplant day Density of <ju dgement> | Sasanisiki Bud May 5 th 19.6 stocks/m² | Hitomebore Bud May 9 th 19.7 stocks/m² |

| Amount of compost | Element of this materials inside | Basal Fertilizer(Element Kg/a) | | | The additional fertilizer (lt is 7/15 and is 7/29 during the first year 7/21 and second of year) | | |
|---|---|---|---|---|---|---|---|
| | (Kg/a) NP₂O₅K₂O | N (The Year) First /Second | P₂ O₅ First /Second | K₂ O First /Second | N First /Second | P₂ O₅ First /Second | K₂ O First /Second |
| Tne control | (Keikaru4Kg/a) | 4.0    4.0 | 9.0    12.0 | 6.0 10.0 | 0.1    0.11 | 0    0 | 0.1 0.11 |
| 250Kg district | 2.1    5.3    2.2 | 1.0    2.0 | 3.0    12.0 | 2.0 10.0 | 0.1    0.11 | 0    0 | 0.1 0.11 |
| 500Kg district | 4.7    11.9    4.9 | 1.0    2.0 | 3.0    12.0 | 2.0 10.0 | 0.1    0.11 | 0    0 | 0.1 0.11 |

Table 15 (The eye 1.7mm and numerical values are average values of two reams)

| Item / District | | All weight (Kg/a) | Weight of straw (Kg/a) | Weight of puri--fied grain (Kg/a) | Energy brown rice weight index | The rubbi--sh US Weight (Kg/a) | Number of ears /m² | One ear number of paddies() | Number of paddi--es ×10³ | Percentage (%) | Brown rice 1000 (g) |
|---|---|---|---|---|---|---|---|---|---|---|---|
| The control | | 150 | 67.0 | 77.9 | 59.1 | 100 | 480 | 77.7 | 37.3 | 80.7 | 19.6 |
| Test area | 250Kg | 146 | 64.6 | 77.5 | 60.0 | 102 | 487 | 75.0 | 36.5 | 81.7 | 19.6 |
| | 500Kg | 141 | 62.1 | 75.1 | 57.6 | 98 | 475 | 73.5 | 34.9 | 84.2 | 19.7 |
| Control district | | 131 | 49.7 | 70.5 | 53.4 | 100 | 463 | 73.9 | 34.2 | 67.5 | 21.2 |
| Test area | 250Kg区 | 128 | 50.6 | 69.8 | 52.4 | 98 | 504 | 69.8 | 35.2 | 72.7 | 21.1 |
| | 500Kg区 | 132 | 50.0 | 71.2 | 52.7 | 98 | 500 | 73.2 | 36.6 | 69.2 | 21.1 |

Table 16

| Area | Paragraph (Goods) | | Size distribution of brown rice grain — Percentage of thickness of grain (%) 1.7(mm² turning point) | | | | | | Claeaned rice taste element amoun | | Brown rice quality | senses examinations (It is stand ardoft he cont rast dis tric t). | | | | | |
|---|---|---|---|---|---|---|---|---|---|---|---|---|---|---|---|---|---|
| | | | >2.2 mm | 2.2 ~ 2.1 mm | 2.1 ~ 2.0 mm | 2.0 ~ 1.9 mm | 1.9 ~ 1.8 mm | 1.8 ~ 1.7 mm | The content of Protain (%) | The coatent of Amilose (%) | Investion class | Out line | Sme-ll | Tas-te | Bis-cocity | Hord-ncc | To-tal |
| F i rY se ta r | Control district | | 1.3 | 9.4 | 48.2 | 32.8 | 6.3 | 2.0 | 6.20 | 8.9 | One in | | | | | | - |
| | T ea sr te a | 250Kg area | 1.9 | 11.5 | 47.5 | 30.9 | 6.7 | 1.5 | 5.80 | 8.4 | One un | | | | | | -0.08 |
| | | 500Kg area | 1.7 | 11.0 | 49.7 | 31.6 | 4.8 | 1.2 | 6.27 | 8.6 | One in | | | | | | -0.17 |
| S e cY oe na dr | Control district | | 1.3 | 9.4 | 48.2 | 32.8 | 6.3 | 2.0 | 5.77 | 1.1 | One-on | - | - | - | - | - | - |
| | T ea sr te a | 250Kg area | 1.9 | 11.5 | 47.5 | 30.9 | 6.7 | 1.5 | 5.80 | 1.3 | One in | +0.4 | -0.2 | +0.5 | +0.4 | +0.1 | -0.2 |
| | | 500Kg area | 1.7 | 11.0 | 49.7 | 31.6 | 4.8 | 1.2 | 6.26 | 0.8 | One in | +0.4 | +-0 | +0.1 | +0.1 | +0.3 | -0.1 |

Table 17

| Analysis item / Area / Soil layer | | | | ph (H₂0) | ph (Kcl) | All nitrogen (%) | Effective Phosphorous acid (mg/100g) | C E C (mg/100g) | Exchanged base (mg/100g) CaO | MgO | K₂O |
|---|---|---|---|---|---|---|---|---|---|---|---|
| First year | The control | | Cultivas--thing Fierd | 6.0 | 4.7 | 0.18 | 14.30 | 21.87 | 397 | 50.6 | 25.7 |
| | | | The soil of lower layer | 6.6 | 5.1 | 0.20 | 4.22 | 22/17 | 428 | 60.2 | 19.1 |
| | Test area | 250 | Cultivas--thing Fierd | 6.2 | 4.9 | 0.21 | 15.21 | 24.27 | 405 | 47.9 | 28.6 |
| | | area | The soil of lower layer | 6.9 | 5.4 | 0.14 | 2.75 | 22.67 | 490 | 71.0 | 21.2 |
| | | 500 | Cultivas--thing Fierd | 6.3 | 5.2 | 0.17 | 13.75 | 21.87 | 523 | 52.2 | 32.6 |
| | | area | The soil of lower layer | 7.1 | 5.7 | 0.11 | 1.65 | 22.47 | 513 | 74.1 | 27.1 |
| Second year | Control district | | Cultivas--thing Fierd | 6.1 | | 0.16 | 15.2 | | 405 | 77.6 | 43.5 |
| | | | The soil of lower layer | 6.3 | | 0.16 | 13.4 | | 492 | 72.1 | 39.3 |
| | Test area | 250 | Cultivas--thing Fierd | 6.3 | | 0.17 | 16.5 | | 423 | 56.5 | 41.0 |
| | | area | The soil of lower layer | 6.7 | | 0.13 | 11.4 | | 500 | 72.0 | 43.0 |
| | | 500 | Cultivas--thing Fierd | 6.5 | | 0.16 | 17.4 | | 488 | 47.4 | 45.5 |
| | | area | The soil of lower layer | 6.8 | | 0.09 | 6.1 | | 531 | 76.4 | 74.5 |

Table 18

| |
|---|
| Cultivation place:Aza-Karima,Oh-aza-nakayazawa,Shikawa, Yama -gun,Fukusima Kobayasi Hiroyuki |
| Cultivation scale:44a Cultivation crops: Barley. |
| Examination area:Refer to the figurbelow. |
| Amount of mature: 13.6Kg/10a |
| Additional fertilizer ・・・ Ammonium sulfate (It is 36.3g/10a during the first 30Kg/10a,year second of year |
| Cultivation period: Year first ・・ H3 9.30(sowing)- H4.6.25 (harvest)(about nine months) |
| Year second ・・ H4 9.27(sowing)-H5.6.22(harvest) (about nine months) |

Table 19

| Test area | Cultivation area | This content of material ussed | | Purified barly | | Amount of rubbish crude barly | | Crude barly total amount (Kg) | |
|---|---|---|---|---|---|---|---|---|---|
| | | Year first | Year second | Year first | Year second | Year first | Year second | Year first | Year second |
| A | 10a | 300Kg | 511Kg | 390 | 589 | 58 | 133 | 448 | 722 |
| B | 10a | 600Kg | 511Kg | 390 | 499 | 60 | 132 | 450 | 631 |
| C | 10a | 900Kg | 511Kg | 340 | 466 | 43 | 122 | 383 | 588 |
| D | 13a | 1200Kg | 717Kg (14a) | 433 | 658 | 51 | 134 | 484 | 792 |
| E | 1a | 0Kg | | 19 | | 2 | | 21 | |
| Integrated meter | 44a | 3000Kg | 2250Kg | 1573 | 2212 | 214 | 521 | 1786 | 2733 |

Table 20

| Comparison Item / Comparison value | | Effect of \<giving\> | | Effect of operation | |
|---|---|---|---|---|---|
| | | Con--trol | Test | Year first | Year second |
| Amount of Purifted barley | Kg | 190 | 361 | 1573 | 2212 |
| | Index | 100 | 190 | 100 | 141 |
| Amount of wasted barly | Kg | 20 | 49 | 214 | 521 |
| | Index | 100 | 245 | 100 | 243 |
| Total amount | Kg | 210 | 410 | 1786 | 2733 |
| | Index | 100 | 195 | 100 | 153 |

Table 21

| Item | Contents |
|---|---|
| Cultivation scale | Alley one district 12 m$^2$ |
| Soil | Grayish low ground soil (of rice field conversion field) |
| Contral | No addition of present invention |
| The present invention added | Addition of present invention 1t/10a |
| Number of plants | 31 plants / 1 experimentol area |
| Species | Beniazuma |
| Amount of fertilizer | 16Kg/10a Basel fertilizer (8-8-8) |
| | 16Kg/10a Additional fertilizer (8-8-8) |
| | $K_2$ ($SO_4$)8Kg/10a |
| Cultivation period | 5/25-10/19 (147 days) |
| Amount harvested | Measurement of the weight of the harvest in all rhe districts |
| Quality harvested | Amount of water and starch |

Table 22

| | Experimental area | Control | | The present invention added | |
|---|---|---|---|---|---|
| Item | | Amount of tained | Index | Amount of tained | Index |
| Amount of harvest | Net amount of harvest (Kg/12 m²) | 33.3 | 100 | 43.6 | 131 |
| | Average weight a piece of harvest(g) | 355 | 100 | 495 | 139 |
| | Amount of harvest of tained from 10a | 778 | 100 | 3630 | 131 |
| Quality | Water (%) | 72.3 | 100 | 68.4 | 95 |
| | Amount of starch (%) | 20.9 | 100 | 26.1 | 125 |
| | Amount of carbohydrate (%) | 23.6 | 100 | 27.5 | 117 |
| | Amount of starch of tained from 10a (Kg) | 581 | 100 | 948 | 163 |

Table 23

| Purpose of exbilition | Investigation of the effect of present invention against soil disease of "Long potate" (especially, root disease). |
|---|---|
| Experimental | 1990 ~ 1992 |
| The place of experiment | Kosikakezawa 14-1, Itunohe-cho, Sannohe-gun, Aomori Keiya Kawamura |
| Aolvisers | Itunohe-cho agricultural cooperative association Hitoshi Ookubo<br><br>Hachinohe agricultural improvement popularization Takashi Yamada (Present Towada agricultural improvement popularization ) |

Table 24

| Area name | Scale | Year first | Year second | Year third |
|---|---|---|---|---|
| The control | 10a | GM without supplied (farmer habitual practice compost) | GM1,700Kg supplied<br>All layers 945Kg<br>ditch 755Kg | GM without supplied (farmer habitual practice compost) |
| Examination district | 10a | GM3, 390Kg<br>All layers 1,800Kg<br>1,500Kg | GM1, 700Kg<br>All layers 945Kg<br>755Kg | GM 900Kg<br>All layers 270Kg<br>630Kg |

Table 25

| Kind | "Gankubimijika" |
|---|---|
| Seed disinfection | 10 part of Ben rate T20 100 time liquid soaking |
| Kind of seed | One child born within a year of another · · · 130g-160g(year first), 150g (year second), and 100g(year third) |
| Planting | Jun 2 3 th (Year first) 、May 2 7 th (Year second) , May 2 7 th (Year third) |
| Density of <judgment> 植 | 27cm (3,367/10a) between stocks 110cm in levee width |

Table 26

| (10a/Kg) | | N | $P_2O_5$ | $K_2O$ | Note | |
|---|---|---|---|---|---|---|
| Basel fertilizer | Year first | 12.0 | 12.0 | 12.0 | CDU 555 | 80Kg/10a |
| | Year second | 18.0 | 18.0 | 18.0 | CDU 555 | 120Kg/10a |
| | Year third | 18.0 | 18.0 | 18.0 | CDU 555 | 120Kg/10a |
| Additional supply | Year first | 9.8 | 2.8 | 14.0 | S 440 | 70Kg/10a (7/10,7/24,8/6) |
| | Year second | 14.0 | 4.0 | 20.0 | S 440 | 140Kg/10a (7/20,8/6) |
| | Year third | 14.0 | 4.0 | 20.0 | S 440 | 140Kg/10a (7/20,8/6) |
| Total | Year first | 21.8 | 14.8 | 26.0 | (*yohrin*) 200Kg/10a | |
| | Year second | 32.0 | 22.0 | 38.0 | (*yohrin*) 200Kg/10a | |
| | Year third | 32.0 | 22.0 | 38.0 | (*yohrin*) 200Kg/10a | |

Table 27

| | Year first | Year second | Year third |
|---|---|---|---|
| Kind of weeding medicine | | Mythet (6/15) | Mythet (6/15) |
| | Mythet | Rolox (6/15) | Rolox (6/15) |
| Net profit frequency | Two times of net profit | Two times of net profit | Two times of net profit |
| Pest control medicine Use frequency | Five times (7/5,19,30,8/11,23) | Three times | Three times |

Table 28

| | Amount of sunshiny | Amount of rainfall | Temperature | Damage such as typhoons | Others |
|---|---|---|---|---|---|
| Year first | | Small | Height | | Growing is smooth. |
| Year second | Small | | | Having | Growing is defective. |
| Year third | | Small | Height | | Growing is smooth. |

Table 29
(The investigation number:each of 20 districs and numerrical values:% and year second:Examination district with both districts).

| Year | Standard | The Control | | | | | | Test area | | | | | |
|---|---|---|---|---|---|---|---|---|---|---|---|---|---|
| | | A | B | C | A- | B | D | A | B | C | A- | B | D |
| Year first | 4 L | | | | | | | 30 | | | 5 | | |
| | 3 L | 10 | | | | | | 20 | | | | | |
| | 2 L | 10 | 20 | 10 | | 5 | | | 20 | | | | |
| | L | 10 | 15 | | | | | 10 | 10 | 5 | | | |
| | M | | 20 | | | | | | | | | | |
| | S | | | | | | | | | | | | |
| | Total | 30 | 55 | 10 | | 5 | | 60 | 30 | 5 | 5 | | |
| Years record | 4 L | 5 | | | | | | | | | | 5 | |
| | 3 L | 5 | 10 | | 20 | | | | | 5 | | | 5 |
| | 2 L | 25 | | | 20 | | | 15 | 25 | 5 | | | |
| | L | | 15 | | | | | | 20 | | 5 | 5 | |
| | M | | | | | | | 5 | | | | | |
| | S | | | | | | | 5 | | | | | |
| | Total | 35 | 25 | | 40 | | | 25 | 45 | 10 | 5 | 10 | 5 |
| Year third | 4 L | | | | | | | | | | | | |
| | 3 L | | | | | | | | | | | | |
| | 2 L | | 5 | | 5 | | 5 | 5 | 20 | | | | |
| | L | 10 | 10 | | | 5 | | | 30 | 35 | | | |
| | M | | 20 | 5 | | | 5 | | 10 | | | | |
| | S | 5 | 5 | 5 | | | | | | | | | |
| | Total | 15 | 40 | 10 | 5 | 5 | 10 | 5 | 60 | 35 | | | |

29

Table 30

| Item / Number of serial cultivation | | Moisture (%) | Index | Content of starch (%) | Index | Senior standard goods (%) | Index |
|---|---|---|---|---|---|---|---|
| First area | The control | 82.3 | 100 | 59.9 | 100 | 65 | 100 |
| | Test area | 81.7 | 99 | 67.0 | 112 | 90 | 138 |
| Second year | The control | 86.1 | 100 | 61.7 | 100 | 60 | 100 |
| | Test area | 86.1 | 100 | 63.2 | 102 | 60 | 100 |
| Third year | The control | 78.1 | 100 | 78.1 | 100 | 25 | 100 |
| | Test area | 76.4 | 98 | 76.4 | 10 5 | 55 | 220 |

30

Table 31

| Item | Content of cultivation |
|---|---|
| Cultivation period | (*purantah*)(14×57×17cm) and 3 reams |
| Kind | Green soybeans (beauty garden green). |
| Soil | Surface humus quality pozzuo lana soil lower soil |
| Soil improvement | (Bamikyurait)is added.20% of the capacity of the soil 5% equivalent phosphorous acid absorption coefficient addition of heavy s corch phosphorous |
| Control district | No materials districts and the compost districts(It is work in each 2t/10a). |
| Examination district | (materials), (work) |
| Amount of manure | 8Kg/10a of Ube transformation(8-8-8) |
| Amount of sowing | Three place×five/(*purantah*) |
| Thinning out | Three place×3 stocks(nine stocks)/(*purantah*) |
| Root nobule investiganation | (harvest), (root), (water), (soil) is removed every one stock. Weight measurement after drying 70° C |
| Cultivation period | The first work: 8/23-10/18 (50) |
| | The second work: 10/25-1/06 (71) |
| | The third work: 1/07-3/22 (75) |
| | The fourth work: 5/10-7/17 (67) |

Table 32                                                                (Weight : g／9 Strain)

| | | 連数 | The first work | | | The second work | | | The third work | | | Four works | | |
|---|---|---|---|---|---|---|---|---|---|---|---|---|---|---|
| | | | 3 be-aus Weight | 2 be-aus Weight | Total | 3 be-aus Weight | 2 be-aus Weight | Total | 3 be-aus Weight | 2 be-aus Weight | Total | 3 be-aus Weight | 2 be-aus Weight | Total |
| The control | No material | 1 | 33 | 113 | 146 | 17 | 58 | 75 | 31 | 92 | 123 | 25 | 104 | 129 |
| | | 2 | 47 | 89 | 136 | 24 | 62 | 86 | 42 | 92 | 134 | 20 | 81 | 101 |
| | | 3 | 20 | 104 | 124 | 25 | 89 | 114 | 22 | 81 | 103 | | | |
| | | Average | 33 | 102 | 135 | 22 | 70 | 92 | 32 | 88 | 120 | 23 | 93 | 115 |
| | | Index | 100 | 100 | 100 | 100 | 100 | 100 | 100 | 100 | 100 | 100 | 100 | 100 |
| | Compost | 1 | 48 | 96 | 144 | 32 | 74 | 106 | 51 | 65 | 116 | 58 | 101 | 159 |
| | | 2 | 24 | 104 | 128 | 56 | 68 | 124 | 62 | 69 | 131 | 39 | 113 | 152 |
| | | 3 | 24 | 109 | 133 | 26 | 81 | 107 | 51 | 62 | 113 | | | |
| | | | 32 | 101 | 135 | 38 | 74 | 112 | 55 | 65 | 120 | 49 | 107 | 156 |
| | | | 97 (100) | 99 (100) | 100 (100) | 173 (100) | 106 (100) | 122 (100) | 172 (100) | 74 (100) | 100 (100) | 213 (100) | 115 (100) | 136 (100) |
| Test area | Matthesia infection | 1 | 35 | 101 | 136 | 54 | 79 | 133 | 42 | 100 | 142 | 60 | 115 | 175 |
| | | 2 | 39 | 98 | 137 | 46 | 84 | 130 | 28 | 77 | 105 | 21 | 80 | 101 |
| | | 3 | 38 | 92 | 130 | 83 | 80 | 163 | 26 | 89 | 115 | | | |
| | | | 37 | 98 | 135 | 61 | 81 | 142 | 33 | 89 | 121 | 41 | 98 | 138 |
| | | | 112 (116) | 96 (97) | 100 (100) | 277 (161) | 116 (109) | 154 (127) | 103 (60) | 101 (137) | 101 (101) | 178 (84) | 107 (92) | 120 (88) |

Table 33

| Item | Content | | |
|---|---|---|---|
| Cultivation scaie | Made of plastic 5号 Deep pot (surface area154cm 2,depth 12cm,Real capacity 1litter) | | |
| Number of reams | Five reams | | |
| Soil xamination district | Akadamatuti    (small)<br>Control area(no materials Use andthose supplied with compost it and 2t per work)<br>Test area(materials Use andthose supplied with compost it and 2t per work) | | |
| Cultivation frequency | The first work | The second work | The third work |
| Addition al fertiliz er — Pasic supply | 20Kg/10a of transformation<br>                          (8-8-8)<br>(8-8-8)20Kg/10a,Ammorium<br>Night Rait(N)      20Kg/10a | 40Kg/10a of Ube transformation<br>          (8-8-8)<br>None | 20Kg/10a of Ube transformation<br>(8-8-8)20Kg/10a |
| Addition al fertiliz er — Total | (8-8-8)40Kg/10a,Ammorium<br>Night Rait(N)      20Kg/10a | (8-8-8)40Kg/10a | (8-8-8)40Kg/10a |
| Kind | Homely woman (low temperature) | Trial(low temperature) | Slowness(   is taken in (*paruk* ) in summer) |
| Sowing density | thinning out 20/one bowl→<br>              5stock/one bowl | thinning out 10/one bowl→5 stock/one bowl | thinning out 6/one bowl→<br>              3 stock/one bowl |
| Acid degree correction | None | None | CaCO₃ 160Kg/10a (contrast district) |
| Medicine | One time of liquid of marathon 2000 times scatter<br>Two time of liquid of (*keruse n*) 1500 times scatter | None<br><br>None | None<br><br>None |
| Cultivation period | 1/27-3/21 (while 66 days) | 4/21-5/16<br>(while 33 days) | 5/27-7/19 (while 47 days) |

Table 34

| Area | Amount | Number of Reams | The first work | | The second work | | The third work | |
|---|---|---|---|---|---|---|---|---|
| | | | beight grass (cm) | Life weight (g/5 stock) | Beight grass (cm) | Life weight (g/5 stock) | Beight grass (cm) | Life weight (g/5 stock) |
| The control area | No materials area | 1 | 18.6 | 68.9 | 8.2 | 7.1 | 8.8 | 23.5 |
| | | 2 | 20.5 | 68.9 | 5.2 | 2.9 | 10.3 | 21.2 |
| | | 3 | 19.3 | 67.2 | 9.2 | 7.9 | 13.2 | 28.0 |
| | | 4 | 21.2 | 76.5 | 8.4 | 6.7 | 12.2 | 26.4 |
| | | 5 | 20.2 | 78.0 | 7.9 | 5.6 | 13.5 | 29.4 |
| | | Average | 20.0 | 71.9 | 7.8 | 6.0 | 11.6 | 25.8 |
| | | Index | 100 | 100 | 100 | 100 | 100 | 100 |
| | compost area | 1 | 19.5 | 77.0 | 14.0 | 24.6 | 15.7 | 50.3 |
| | | 2 | 20.8 | 83.1 | 15.6 | 34.5 | 14.8 | 46.3 |
| | | 3 | 20.9 | 60.9 | 12.3 | 17.6 | 17.5 | 57.7 |
| | | 4 | 20.8 | 87.0 | 15.4 | 29.1 | 15.5 | 43.2 |
| | | 5 | 21.9 | 76.4 | 13.3 | 11.6 | 15.5 | 47.4 |
| | | Average | 20.8 | 76.9 | 14.1 | 23.5 | 15.8 | 25.8 |
| | | Index | 104 (100) | 107 (100) | 181 (100) | 392 (100) | 136 (100) | 190 (100) |
| Test area | Materials of this invetion area | 1 | 20.3 | 67.5 | 15.0 | 32.0 | 17.8 | 68.5 |
| | | 2 | 20.1 | 69.1 | 14.2 | 29.0 | 21.1 | 89.7 |
| | | 3 | 18.6 | 65.8 | 16.5 | 37.5 | 20.0 | 81.9 |
| | | 4 | 19.6 | 79.1 | 19.0 | 54.5 | 20.7 | 89.2 |
| | | 5 | 21.0 | 82.1 | 19.4 | 49.9 | 18.8 | 90.5 |
| | | Average | 19.9 | 74.5 | 16.8 | 40.6 | 19.7 | 84.0 |
| | | Index | 100 (96) | 104 (97) | 215 (119) | 677 (173) | 170 (125) | 326 (171) |

(note)height gurss is an average value of 5 or 3. As
for index column () of the compost district,
and the examination district are compared.

Table 35

| Item | Content of cultivation |
|---|---|
| Cultivation period | (*purantah*)(14×57×17cm) and 3 reams |
| Kind | Green soybeans (beauty garden green). |
| Soil | Surface humus quality pozzuo lana soil lower soil |
| Soil improvement | (Bamikyurait)is added, 20% of the capacity of the soil 5% equivalent phosphorous acid absorption coefficient addition of heavy s corch phosphorous |
| Control district | No materials districts and the compost districts (It is work in each 2t/10a). |
| Examination district | (materials), (work) |
| Amount of manure | 8Kg/10a of Ube transformation(8-8-8) |
| Amount of sowing | Three place×five/(*purantah*) |
| Thinning out | Three place×3 stocks(nine stocks)/(*purantah*) |
| Root nobule investiganation | harvest), (root), (water), (soil) is removed every one stock. Weight measurement after drying 70° C |
| Cultivation period | The first work: 8/23-10/18 (50) |
| | The second work: 10/25-1/06 (71) |
| | The third work: 1/07-3/22 (75) |
| | The fourth work: 5/10-7/17 (67) |

Table 36

| Area | Frequency | Number of reams | The first work | The second work | The third work | The fourth work | Four serial cultivation total | |
|---|---|---|---|---|---|---|---|---|
| The control | No materials area | 1 2 3 | 3.91 2.99 3.99 | 0.81 1.05 1.51 | 1.08 1.48 2.44 | 0.80 0.40 | Total weight (99 stocks) | 20.46 |
| | | Average | 3.63 | 1.12 | 1.67 | 0.60 | Average (nine stocks) | 1.86 |
| | | Index | 100 | 100 | 100 | 100 | Index | 100 |
| | Compost area | 1 2 3 | 3.45 3.72 2.51 | 2.29 2.38 1.47 | 0.89 0.96 1.21 | 0.66 0.88 | Total weight (99 stocks) | 20.42 |
| | | Average | 3.23 | 2.05 | 1.02 | 0.77 | Average (nine stocks) | 1.86 |
| | | Index | 89 | 183 | 61 | 128 | Index | 100 |
| Test area | Material of this inven-tion area | 1 2 3 | 3.05 4.17 2.98 | 5.60 2.86 5.07 | 2.03 0.22 0.73 | 1.23 1.02 | Total weight (99 stocks) | 28.96 |
| | | Average | 3.40 | 4.51 | 0.99 | 1.13 | Average (nine stocks) | 2.63 |
| | | Index | 94 | 403 | 59 | 188 | Index | 141 |

Table 37

| Investigation item / cultivation years | Number of investigation stocks ( ) | Number of attack stocks () | Attack stock rate (%) | Level of disease ( ) ( 1) | | | | | Disease degree (2) |
|---|---|---|---|---|---|---|---|---|---|
| | | | | 0 | 1 | 2 | 3 | 4 | |
| Control area — Year first | 20 | 11 | 55 | 9 | 10 | 1 | 0 | 0 | 15.0 |
| Control area — Year second | 20 | 1 | 5 | 19 | 1 | 0 | 0 | 0 | 1.3 |
| Control area — Year third | 20 | 7 | 35 | 13 | 5 | 2 | 0 | 0 | 11.3 |
| Test area — Year first | 20 | 1 | 5 | 19 | 1 | 0 | 0 | 0 | 1.3 |
| Test area — Year second | 20 | 0 | 0 | 20 | 0 | 0 | 0 | 0 | 0 |
| Test area — Year third | 20 | 0 | 0 | 20 | 0 | 0 | 0 | 0 | 0 |

## Claims

1. A fermentative product characterized by;
   consisting of a reaction product obtained through a reaction between a putrefactive waste and an additive whose main component is highly activated calcium oxide,
   containing microorganisms surviving under highly alkaline environment with pH of 12 or more,
   the microorganisms consisting of bacteria in 3 groups, filamentous fungi and 90A1 bacterium,
   the first group of the bacteria starting its proliferation at pH of 11 to less than 12, the second group of the bacteria preferably growing at pH of around 11 to 9 and the third group of the bacteria preferably growing at ph of less than around 9,
   at least the first group of the bacteria being more in the number through proliferation thereof than they were, i.e., those in the same group which were contained in the reaction product.

2. The fermentative product characterized by its pH maintained at around 8 through a proliferation of said first to third groups of the bacteria.

3. The fermentative product of claim 1 in which the bacterium in said first group is a strain No.GM-4.

4. The fermentative product of claim 1 in which the bacteria in said first group are strain No.GM-4 and GM-5

5. The fermentative product of claim 1 in which the bacterium in said second group is a strain No.GM-5.

6. The fermentative product of claim 1, 3 or 4 in which the bacterium in said second group is a strain No.GM-8.

7. The fermentative product of claim 1, 3 or 4 in which the bacterium in said second group is a strain No.GM-9.

8. The fermentative product of claim 1, 3 or 4 in which the bacterium in said second group is a strain No.90-4.

9. The fermentative product of claim 1, 3 or 4 in which the bacteria in said second group are one of combinations among strain No.GM-5, GM-8, GM-9 and 90-4.

10. The fermentative product of one of claims 1 to 9 in which the bacterium in said third group is a strain No.90-7.

11. The fermentative product of one of claims 1 to 9 in which the bacterium in said third group is a strain No.90-11.

12. The fermentative product of one of claims 1 to 9 in which the bacterium in said third group is a strain No.90-1.

13. The fermentative product of one of claims 1 to 9 in which the bacterium in said third group is a strain No.90-8.

14. The fermentative product of one of claims 1 to 9 in which the bacterium in said third group is a strain No.90-4.

15. The fermentative product of one of claims 1 to 9 in which the bacterium in said third group is a strain No.90-2.

16. The fermentative product of one of claims 1 to 9 in which the bacterium in said third group is a strain No.90-3.

17. The fermentative product of one of claims 1 to 9 in which the bacterium in said third group is a strain No.90-10.

18. The fermentative product of one of claims 1 to 9 in which the bacteria in said third group are one of combinations among strains No.90-7, 90-11, 90-1, 90-8, 90-4, 90-3, 90-2, and 90-10.

19. The fermentative product of one of claims 1 to 18 in which said filamentous fungi is a strain No. 90-F1.

20. Method for producing the fermentative product is characterized by the steps of;

   causing a vigorous hydration reaction by adding an additive whose main component is highly activated quick lime to the putrefactive waste,
   suspending the putrefaction of the reaction product by the co-actions of the reaction heat generated from this hydration reaction and alkaline sterilization under the highly alkaline environment, while allowing existence of the microorganisms which are detectable in status of durability and can survive in highly alkaline condition, the microorganisms consisting of the bacteria in 3 groups, filamentous fungi and 90A1 bacterium, the first group of the bacteria starting its proliferation at pH of 11 to less than 12, the second group of the bacteria preferably growing at pH of around 11 to 9 and the third group of the bacteria preferably growing at pH of less than around 9,
   supplying water and air at a suitable period for the reaction product thereby utilizing the bacteria to effect sequential decline in pH value,
   and causing proliferations of filamentous fungi and 90A1 bacterium which have been in status of durability and survived in the reaction product with the decline in pH for alkaline fermentation of the reaction product.

21. The method for producing the fermentative product of claim 20 in which the produced fermentative product is mixed into said putrefactive waste, thereby carrying out water control.

22. The method for producing the fermentative product in which calcium ion in calcium oxide is dissociated by said hydration reaction while lower fatty acids such as formic acid are produced utilizing amino acid contained in said putrefactive waste
   and calcium salt is produced utilizing said dissociated calcium ion and acids such as amino acid and fatty acids including lower fatty acids such as formic acid originally contained in the putrefactive waste.

23. The method for producing the fermentative product of one of claims 20 to 22 in which the reaction product just after said hydration reaction is in the highly alkaline environment with pH of 12 to 13.

24. The method for producing the fermentative product of one of claims 20 to 22 in which the bacterium in said first group is a strain No. GM-4.

**25.** The method for producing the fermentative product of one of claims 20 to 23 in which the bacteria in said first group are strain No. GM-4 and GM-5.

**26.** The method for producing the fermentative product of one of claims 20 to 24 in which the bacterium in said second group is a strain No. GM-5.

**27.** The method for producing the fermentative product of one of claims 20 to 25 in which the bacterium in said second group is a strain No. GM-8.

**28.** The method for producing the fermentative product of one of claims 20 to 25 in which the bacterium in said second group is a strain No. GM-9.

**29.** The method for producing the fermentative product of one of claims 20 to 25 in which the bacterium in said second group is a strain No. 90-4.

**30.** The method for producing the fermentative product of one of claims 20 to claim 25 in which the bacteria in said second group are one of combinations among strains No. GM-5, GM-8, GM-9 and 90-4.

**31.** The method for producing the fermentative product of one of claims 20 to 30 in which the bacterium in said third group is a strain No. 90-7.

**32.** The method for producing the fermentative product of one of claims 20 to 30 in which the bacterium in said third group is a strain No. 90-11.

**33.** The method for producing the fermentative product of one of claims 20 to 30 in which the bacterium in said third group is a strain No. 90-1.

**34.** The method for producing the fermentative product of one of claims 20 to 30 in which the bacterium in said third group is a strain No. 90-8.

**35.** The fermentative product of one of claims 20 to 28 in which the bacterium in said third group is a strain No. 90-4.

**36.** The method for producing the fermentative product of one of claims 20 to 30 in which the bacterium in said third group is a strain No. 90-2.

**37.** The method for producing the fermentative product of one of claims 20 to 30 in which the bacterium in said third group is a strain No. 90-3.

**38.** The method for producing the fermentative product of one of claims 20 to 30 in which the bacterium in said third group is a strain No. 90-10.

**39.** The method for producing the fermentative product of one of claims 20 to 30 in which the bacteria in said third group are one of combinations among strains No. 90-7, 90-11, 90-1, 90-8, 90-4, 90-3, 90-2, and 90-10.

**40.** The method for producing the fermentative product of one of claims 20 to 30 in which said filamentous fungi is a strain No. 90-F1.

FIG. 1

Time of Hydration reaction (second)

(Cao 20g + water of 100ml, initial temperature of water 20℃)

EP 0 745 573 A1

FIG. 2

Ratio of experimental reaction temperature against theoretical value(%)

Time of Hydration reaction (second)

(Cao 20g + water of 100ml, reduced temperature against theoritical reaction heat 46 ℃)

41

FIG. 3

○:Volcanic ash soil(vegetable field of Kanto-rom surface layer soil)

●:Deposited soil by river fluid conveying(sandy soil in Ohi-river fulid

  depositing soil area, soil for gardening in the facility)

△:Mineral soil(from Iwata-shi,Shizuoka-Pref.; viscos yellow soil of

  fine paraticles, prime soil)

FIG. 4

Amount of material to be supplied (weight t/cultivated soil 10a)

EP 0 745 573 A1

FIG. 5

FIG. 6

EP 0 745 573 A1

FIG. 7

the number of days of fermentation

FIG. 8

the number of days of fermentation

EP 0 745 573 A1

FIG. 9

N ←—————|—————

Material of this invention    500kg/10a area
                    (4a)

                                        Water outlet

Material of this invention
250kg/10a area

                        The control  (2a)

Water inlet    (2a)

48

FIG. 10

This area was marged into D area

⊣⊢— 1／500

The control

High

To the mountain

E area

Material of this invention

1,500Kg area (13a)

D area

Material of this invention

900Kg area (10a)

C area

Road

Material of this invention

600Kg area (10a)

B area

ountains and Forests

Material of this invention

300Kg area (10a)

A area

To the foot

Low

## INTERNATIONAL SEARCH REPORT

International application No.

PCT/JP94/02155

**A. CLASSIFICATION OF SUBJECT MATTER**

Int. Cl$^6$  C05F11/08, C02F11/02, B09B3/00, C12P1/00

According to International Patent Classification (IPC) or to both national classification and IPC

**B. FIELDS SEARCHED**

Minimum documentation searched (classification system followed by classification symbols)

Int. Cl$^6$  C05F1/00-17/02, C02F11/00-11-20, B09B3/00, C12P1/00-1/06

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, A, 2-153889 (Moriie Nakajima)<br>June 13, 1990 (13. 06. 90) (Family: none) | 1-40 |
| A | JP, A, 56-69291 (Yoshifusa Monma)<br>June 10, 1981 (10. 06. 81) (Family: none) | 1-40 |
| A | JP, A, 56-161896 (Director General, Agency of Industrial and Science and Technology),<br>December 12, 1981 (12. 12. 81)<br>& US, A, 4354936 | 1-40 |
| A | JP, A, 62-195289 (Director General, Agency of Industrial Science and Technology),<br>August 28, 1987 (28. 08. 87) (Family: none) | 1-40 |
| A | JP, A, 62-97698 (Haward E. Worn),<br>May 7, 1987 (07. 05. 87)<br>& EP, A1, 220647 | 1-40 |

| ☒ | Further documents are listed in the continuation of Box C. | ☐ | See patent family annex. |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| March 1, 1995 (01. 03. 95) | March 20, 1995 (20. 03. 95) |

| Name and mailing address of the ISA/ | Authorized officer |
|---|---|
| Japanese Patent Office | |
| Facsimile No. | Telephone No. |

Form PCT/ISA/210 (second sheet) (July 1992)

50

## INTERNATIONAL SEARCH REPORT

International application No.

PCT/JP94/02155

C (Continuation).   DOCUMENTS CONSIDERED TO BE RELEVANT

| Category* | Citation of document, with indication, where appropriate, of the relevant passages | Relevant to claim No. |
|---|---|---|
| E, X | JP, A, 7-2589 (Mitsuyo Kimura), January 6, 1995 (06. 01. 95) (Family: none) | 1-40 |

Form PCT/ISA/210 (continuation of second sheet) (July 1992)